# EUROPÄISCHE PATENTANMELDUNG

(11) **EP 4 789 753 A1**
(43) Veröffentlichungstag der Anmeldung: **12.08.2026**
(21) Anmeldenummer: 26166327.2
(22) Anmeldetag: 08.12.2017
(51) Int. Cl.: B01D 65/02, B01D 67/00, B01D 71/44, B01D 71/68, B01D 69/02, B01D 69/08

(54) **HOHLFASERMEMBRAN MIT VERBESSERTER TRENNLEISTUNG UND HERSTELLUNG EINER HOHLFASERMEMBRAN MIT VERBESSERTER TRENNLEISTUNG**

(30) Priorität: 09.12.2016 DE 102016224627
(62) Dokumentnummer(n) der früheren Anmeldung(en) nach Art. 76 EPÜ: 17821510.9 / 3 551 321
(71) Anmelder: Fresenius Medical Care Deutschland GmbH, 61352 Bad Homburg (DE)
(72) Erfinder: KELLER, Torsten, 61352 Bad Homburg (DE); PAUL, Michael, 61352 Bad Homburg (DE); SANDER, Roland, 61352 Bad Homburg (DE)
(74) Vertreter: Ricker, Mathias

(57) **Zusammenfassung**

Die Erfindung betrifft eine Hohlfasermembran aufweisend eine hohe Zurückhaltung im hohen Molekulargewichtsbereich, eine hohe Abtrennleistung (Clearance) im Mittelmolekulargewichtsbereich, weiter aufweisend wenigstens ein Material auf Polysulfonbasis und wenigstens ein Polymer auf Basis von Polyvinylpyrrolidon, weiter aufweisend eine Porosität von 77,5% bis 82% und einen Siebkoeffizienten für Dextran mit dem Molekulargewicht von 10.000 g/mol von 0,42 bis 0,75.

## Beschreibung

### Thema der Erfindung

Die Erfindung betrifft Hohlfasermembranen, aufweisend ein Membranmaterial auf Basis von Polysulfon und Polyvinylpyrrolidon, die verbesserte Trenneigenschaften aufweisen, insbesondere die eine bessere Abtrennleistung von Substanzen im Mittelmolekulargewichtsbereich und eine verbesserte Trennschärfe gegenüber der Abtrennung von Substanzen im hohen Mittelmolekulargewichtsbereich aufweisen.

Darüber hinaus betrifft die Erfindung ein Verfahren zur Herstellung von Hohlfasermembranen aufweisend ein Membranmaterial auf Basis von Polysulfon und Polyvinylpyrrolidon.

Weiterhin betrifft die Erfindung einen Hohlfasermembranfilter mit Hohlfasermembranen, mit homogenen Permeationseigenschaften.

Insbesondere betrifft die Erfindung ein Sterilisationsverfahren von Hohlfasermembranen, die ein Membranmaterial auf Polysulfonbasis und Polyvinylpyrrolidon aufweisen.

### Hintergrund der Erfindung

Hohlfasermembranen finden in der Aufreinigung von Flüssigkeiten weiträumig Verwendung. Insbesondere werden Hohlfasermembranen in der Medizintechnik für Wasseraufbereitung und Blutreinigung in der Dialyse von nierenkranken Patienten eingesetzt. Entsprechende Hohlfasermembranen werden in Hohlfasermembranbündeln in Filtermodulen verbaut. Die Herstellung solcher Filtermodule zur Blutreinigung erfolgt im Massenproduktionsmaßstab.

Die für die Blutreinigung verwendeten Hohlfasermembranen bestehen häufig aus Polysulfon (PSU) und Polyvinylpyrrolidon (PVP), da sich diese Materialien als bevorzugt hämokompatibel herausgestellt haben und daher in der Blutbehandlung, insbesondere in der Hämodialyse aus medizinischer Sicht bevorzugt sind. Die grundsätzlichen Prinzipien der Herstellung von Hohlfasermembranen und deren Herstellung sind im Stand der Technik beschrieben:
- Marcel Mulder; Principles of Membrane Technology; Kluwer Academic Publisher 1996; Kapitel III, Preparation of synthetic membranes
- EP 0 168 783
- WO 2007/128440
Nach denen im Stand der Technik beschriebenen Methoden wird eine Spinnlösung bereitgestellt, die ein hydrophobes Material auf Basis von Polysulfon, und ein hydrophiles Polymer auf Basis von Vinylpyrrolidon, insbesondere Polyvinylpyrolidon und ein oder mehrere Lösungsmittel und gegebenenfalls Additive umfasst. Als Lösungsmittel können polare aprotische Lösungsmittel dienen, insbesondere Dimethylacetamid, N-Methylpyrrolidon, Dimethylformamid oder Dimethylsulfoxid. Geringe Zusätze von Additiven, z.B. polare protische Lösungsmittel, z.B. Wasser, können ebenfalls in geringen Anteilen Bestandteile der Spinnlösung sein.

Die Spinnmasse wird über eine kreisförmige Düse extrudiert. Dabei weist die Düse eine innere Bohrung auf, durch die ein Fällmittel geleitet wird und zusammen mit der Spinnmasse koextrudiert wird. Die Spinnmasse wird durch einen die innere Bohrung umgebenden, ringförmigen Spalt zu einem holen Faden extrudiert, in dessen Lumen das Fällmittel geführt wird. Der Spinnfaden wird dann in ein Fällbad eingeleitet, in der sich ein weiteres Fällmittel befindet, so dass sich eine Membranstruktur durch Phaseninversion und Ausfällung zu einer Hohlfasermembran ausbildet. Als Fällmittel dienen Wasser oder Gemische aus protischen und aprotischen Lösungsmitteln, insbesondere Wasser und Dimethylacetamid, N-Metyhlpyrrolidon, Dimethylformamid oder Dimethylsulfoxid. Die erhaltene Hohlfasermembran wird anschließend durch Spülbäder geführt und getrocknet und auf eine Haspel aufgenommen. Von der Haspel können die Hohlfasermembranen in Form von Hohlfaserbündeln entnommen werden. Für den Bau von Hohlfasermembranfiltern werden solche Hohlfasermembranbündel in ein Gehäuse, vorzugsweise ein zylindrisches Gehäuse, eingebracht. Die Enden des Hohlfasermembranbündels werden in eine Vergussmasse eingebettet und die offenen Enden der Hohlfasern freigelegt. Die Vergussmasse bildet einen abdichtenden Bereich zwischen dem Inneren der Hohlfasermembranen, dem Gehäuse und den die Hohlfasermembranen umgebenden Bereich. Es wird dadurch im fertigen Hohlfasermembranfilter ein erster Raum gebildet, der An- und Abströmbereiche der Enden des Hohlfasermembranbündels und auch das Innere der Hohlfasermembranen umfasst. Ein zweiter Raum bildet sich demgemäß durch den Bereich im Zwischenraum zwischen den Hohlfasermembranen und zwischen Gehäusewand und den Hohlfasermembranen. Fluidzugänge am Gehäuse des Hohlfasermembranfilters ermöglichen das Zuführen und Abführen von Flüssigkeiten und Fluiden zu dem ersten und/oder dem zweiten Raum des Hohlfasermembranfilters.

Wenigstens ein Fluidzugang bildet dabei einen Zugang zu dem ersten Raum des Hohlfasermembranfilters. Wenigstens ein Fluidzugang bildet einen Zugang zu dem zweiten Raum des Hohlfasermembranfilters. Gegebenenfalls, je nach Anwendungszweck des herzustellenden Hohlfasermembranfilters, sind weitere Zugänge zu dem ersten und oder zweiten Raum vorgesehen. Hohlfasermembranfilter, die für die extrakorporale Blutbehandlung vorgesehen sind, weisen in der Regel einen ersten und einen zweiten Fluidzugang am ersten Raum des Filtermoduls und einen ersten und einen zweiten Fluidzugang am zweiten Raum des Filtermoduls auf. Fluide, insbesondere Flüssigkeiten oder Gase, können so über den ersten Zugang eines Raums des Hohlfasermembranfilters je nach Strömungsrichtung zu- oder abgeleitet werden, oder über den zweiten Zugang eines Raums des Hohlfasermembranfilters entsprechend der Strömungsrichtung zu- oder abgeleitet werden.

Für Hohlfasermembranfilter, die für medizinische Zwecke vorgesehen sind, insbesondere, die für die Blutbehandlung von nierenkranken Patienten vorgesehen sind, schließt sich an den Herstellprozess der Hohlfasermembranen und dem Filterbau in der Regel ein oder mehrere Spül- und Sterilisationsschritte an, um die Hohlfasermembranen zu reinigen und für den medizinischen Gebrauch zu sterilisieren.

Im Stand der Technik sind entsprechende Verfahren bekannt, bei denen die Hohlfasermembranen in Hohlfasermembranfiltern Spül- und Sterilisationsschritten unterzogen werden. Insbesondere stellt die Hitzesterilisation mit Luft, Wasser oder Wasserdampf in Hohlfasermembranfiltern ein bekanntes Sterilisationsverfahren von Hohlfasermembranen und Hohlfasermembranfiltern dar. Unter Hitzesterilisation ist eine Sterilisation mit Fluiden (beispielsweise Wasser, Luft, Wasserdampf oder Mischungen hieraus) oberhalb einer Temperatur von 100°C zu verstehen. Eine Hitzesterilisation mit überwiegend reinem Wasserdampf wird auch Dampfsterilisation genannt. Ein entsprechendes Verfahren zur Sterilisation von Dialysatoren wird in der DE 39 36 785 C1 beschrieben. Nach dem in der DE 39 36 785 C1 beschriebenen Verfahren werden die Dialysatoren einem Spülprozess und anschließend einem Sterilisationsprozess unterzogen. Bei dem Sterilisationsprozess wird der Dialysator mit auf über 121°C erhitztem Wasser oder Wasserdampf gespült.

Weitere im Stand der Technik bekannte Methoden zur Sterilisation von Filtermodulen ist die Vakuum-Dampf Sterilisation, die Sterilisation mit sterilisierenden Gasen, z.B. Ethylenoxid, die Bestrahlung mit ionisierender oder radikalbildender Strahlung z.B. Elektronenstrahlung oder Gamma-Strahlung.

Es hat sich gezeigt, dass sich die Vakuum-Dampf Sterilisation durch die thermische Wechselbelastung negativ auf die Stabilität der zu sterilisierenden Hohlfasermembranen auswirkt. In der Vakuum-Dampf Sterilisation wechseln sich Verfahrensschritte der Bedampfung und der Evakuierung des Sterilisationsraums ab. Mit jedem Evakuierungsschritt ist ein Absinken der Temperatur der Sterilisationskammer und des Hohlfasermembranfilters weit unterhalb des Bedampfungsschritts nicht zu vermeiden. Der Hohlfasermembranfilter wird dadurch ständig wechselnder Materialausdehnung ausgesetzt. Nachteilig können so beim Vakuum-Dampf Sterilisationsprozess Materialspannungen auftreten. Dies stellt entsprechend aufwendige Anforderungen an die Materialauswahl, Verarbeitung und der Konstruktionen der Hohlfasermembranfilter.

Die Sterilisation mit ionisierender Strahlung, wie z.B. Gamma- oder Elektronenstrahlung ist mit einem hohen apparativen Aufwand verbunden und hat eine hohe zusätzliche Behandlungszeit zur Folge.

Die Sterilisation mit Ethylenoxid erfordert aufgrund der Giftigkeit von Ethylenoxid ebenfalls einen enormen anlagentechnischen Aufwand. Zudem ist nach der Sterilisierung eine lange Phase der notwendigen restlosen Entfernung des Ethylenoxids erforderlich.

Die gemäß der DE 39 36 785 C1 durchgeführte Hitzesterilisation, die durch Spül- und Sterilisationsschritte mit Wasser und/oder Wasserdampf durchgeführt wird, hat sich gegenüber den anderen genannten Sterilisationsmethoden als apparativ und prozesstechnisch überlegen herausgestellt. Insbesondere hat sich die Hitzesterilisation mit Wasser und/oder Wasserdampf in Hinblick auf die Blutkompatibilität der sterilisierten Hohlfasermembranen gegenüber den Sterilisationsmethoden durch Bestrahlung oder durch Begasung als überlegen erwiesen.

Allerdings hat sich auch gezeigt, dass das in der DE 39 36 785 C1 genannte Dampfsterilisationsverfahren für Polysulfonmembranen, die Polyvinylpyrrolidon enthalten, nachteilig auf die Clearance der Hohlfasermembranen auswirken kann. Man geht davon aus, dass die Spülprozesse während des Sterilisationsprozesses auf der Hohlfasermembran befindliches PVP mobilisiert. Durch die Kapillarkräfte der Membranporen wird das mobilisierte PVP in die Poren der Hohlfasermembran gezogen und verengt, oder blockiert den Porenquerschnitt. Dies hat zur Folge, dass im Anwendungsfall ein geringerer Porenquerschnitt für die Filtration zur Verfügung steht. Entsprechend wirkt sich die Ablagerung von PVP in den Poren verschlechternd auf die Permeationseigenschaften der Hohlfasermembranen aus.

Durch eine entsprechend angepasste Herstellung der Hohlfasermembranen wurde versucht, die verschlechterten Permeationseigenschaften der Hohlfasermembranen, die durch das Sterilisationsverfahren hervorgerufen wurden, entgegenzuwirken. Dies hatte zur Folge, dass auch die Porengrößenverteilung der Hohlfasermembran sich nachteilig verbreiterte. Die Porengrößenverteilung von Hohlfasermembranen wirkt sich dabei direkt auf die Trennschärfe der Hohlfasermembran aus.

Weiterhin war nach dem Verfahren der DE 39 36 785 C1 zu erkennen, dass die Hohlfasermembranen im Dialysator durch die bisherigen Verfahren im Sterilisationsprozess mit Wasser oder Wasserdampf aufgrund des vorhandenen PVP's innerhalb eines Hohlfasermembranfilters zusammenhaften können. Dies hatte für die geforderte Sterilität keinen Nachteil. Allerdings zeigte sich, dass derartig zusammengelagerte Hohlfasermembranen durch den Sterilisationsprozess inhomogene Leistungseigenschaften der Hohlfasermembranen innerhalb eines Hohlfasermembranfilters lieferten. Insbesondere stellte sich heraus, dass in Bereichen, in denen Hohlfasermembranen innerhalb eines Hohlfasermembranfilters während der Sterilisation zusammengelagert waren, verschlechterte Permeationseigenschaften gegenüber solchen Bereichen aufwiesen, in denen keine Zusammenlagerung der Hohlfasermembranen vorlag.

Hinsichtlich dieses Aspekts bestand der Anlass die Spül- und Sterilisationsprozesse eines Dampfsterilisationsverfahrens für Hohlfasermembranfilter so weiter zu bilden, dass eine Zusammenlagerung von Hohlfasermembranen innerhalb eines Hohlfasermembranmoduls vermieden werden kann und dass zusätzlich die Verengung oder Blockierung von Poren durch mobilisiertes und abgelagertes PVP vermieden werden kann, gleichzeitig eine hohe Sterilität und Blutkompatibilität der Hohlfasermembranen und der Hohlfasermembranfilter beibehalten werden kann.

Eine wesentliche, in diesem Zusammenhang charakterisierende Eigenschaft von Hohlfasermembranen ist die Clearance. Die Clearance ist eine Maß für die Abtrennleistung einer Hohlfasermembran und gibt an, mit welcher Leistung schädliche Metaboliten bei der Blutreinigung durch eine Behandlung an Hohlfasermembranen entfernt werden können. Methoden zur Bestimmung der Clearance von Hohlfasermembranen sind im Stand der Technik bekannt. Diesbezüglich wird auf die Norm DIN/ EN/ ISO 8637:2014 verwiesen. Dabei wird die Clearance einer Hohlfasermembran gemäß der Norm bestimmt, nachdem aus der Membran ein den entsprechenden Verhältnissen angepasster Testfilter aufgebaut wurde.

In der Entwicklung von Hohlfasermembranen für die extrakorporale Blutbehandlung ist man bestrebt, Hohlfasermembranen mit einer möglichst hohen Abtrennleistung zu entwickeln, um effektive Therapieformen der extrakorporalen Blutbehandlung bereitzustellen.

Insbesondere wird die Abtrennleistung einer Substanz durch die Porosität und die mittlere Porengröße einer Membran beeinflusst. Die Porosität gibt den Anteil des Porenvolumens in einer Membran an. Weist dabei eine Membran ein höheres Porenvolumen als eine Vergleichsmembran auf, so wird ein höherer Stofftransport über die Membranwand hinweg abhängig von der mittleren Porengröße beobachtet.

Insbesondere ist für Therapien in der chronischen extrakorporalen Blutbehandlung eine hohe Abtrennleistung von Plasmaproteinen im Mittelmolekülbereich wünschenswert. Gleichzeitig ist aber eine hohe Rückhaltung von Plasmaproteinen im hohen Molekulargewichtsbereich, wie z.B. Albumin notwendig. Gleichzeitig ist auch eine hohe Hämokompatibilität von Hohlfasermembranen in der extrakorporalen Blutbehandlung anzustreben

### Aufgabe der Erfindung

Es hat sich gezeigt, dass bisherige Methoden in der Herstellung von dampfsterilisierten Hohlfasermembranen auf Polysulfon und Polyvinylpyrrolidon Basis, bedingt durch das Sterilisationsverfahren unter einer begrenzten Abtrennleistung und Trennschärfe, insbesondere einer begrenzten Abtrennleistung von Plasmaproteinen im Mittelmolekulargewichtsbereich bei vorgegebener Zurückhaltung von Albumin leiden.

In einem ersten Aspekt der Erfindung stellte sich daher die Aufgabe, eine Hohlfasermembran mit hoher Abtrennleistung (Clearance) im Mittelmolekulargewichtsbereich und hoher Zurückhaltung im hohen Molekulargewichtsbereich bereitzustellen, wobei die Hohlfasermembran gleichzeitig eine hohe Blutkompatibilität aufweist, wie sie durch ein Hitzesterilisationsverfahren mit Wasser oder Wasserdampf gewährleistet wird.

### Zusammenfassung weiter Aspekte der vorliegenden Anmeldung

Ein zweiter Aspekt der vorliegenden Anmeldung betrifft die Bereitstellung eines Hohlfasermembranfilters, der homogene Leistungseigenschaften bezüglich der Hohlfasermembranen innerhalb eines Hohlfasermembranfilters aufweist.

Ein dritter Aspekt der vorliegenden Anmeldung betrifft ein Verfahren zur Herstellung von Hohlfasermembranen, welches Spül- und/oder Sterilisationsschritte auf Basis von Wasser oder Wasserdampf umfasst, ohne dass die Abtrennleistung der Hohlfasermembranen während der Spül und/oder Sterilisationsschritte verschlechtert wird.

Ein vierter Aspekt der vorliegenden Anmeldung betrifft ein Spül und Sterilisationsverfahren auf Basis von Wasser oder Wasserdampf für Hohlfasermembranen, dass die Leistungseigenschaften der Hohlfasermembranen nicht negativ beeinflusst.

### Zusammenfassung der Erfindung

Die Erfindung ist in den angehängten Ansprüchen dargelegt. In einem ersten Aspekt der Erfindung wurde überraschenderweise gefunden, dass die vorab genannte Aufgabe durch eine Hohlfasermembran nach den Merkmalen von Anspruch 1 gelöst wird. Die Ansprüche 2 bis 9 stellen bevorzugte Ausführungen der Erfindung gemäß des ersten Aspekts dar.

### Detaillierte Beschreibung der Erfindung

Überraschenderweise zeigte sich in einem ersten Aspekt der Erfindung, dass eine Hohlfasermembran mit verbesserter Abtrennleistung im Mittelmolekulargewichtsbereich bereitgestellt werden kann, wenn die Hohlfasermembran wenigstens ein Material auf Polysulfonbasis und wenigstens ein Polymer auf Basis von Vinylpyrrolidon aufweist und die Hohlfasermembran eine Porosität von 77,5% bis 82% und ein Siebkoeffizient für Dextran mit dem Molekulargewicht von 10.000 g/mol von 0,42 bis 0,75 aufweist.

Eine Hohlfasermembran nach dem ersten Aspekt der Erfindung zeichnet sich durch eine hohe Permeabilität im Mittelmolekulargewichtsbereich aus. Insbesondere zeigte sich weiterhin, dass sich derartige Hohlfasermembranen als hämokompatibel erweisen, da sie aus Polymeren auf Polysulfon- und Polyvinylpyrrolidonbasis hergestellt werden können und nach einem Spülverfahren gereinigt und einem Dampfsterilisationsverfahren sterilisiert werden können.

Gemäß dem ersten Aspekt der Erfindung basiert das Material der Hohlfasermembranen auf Polysulfon. Unter einem Polymer auf Polysulfonbasis wird nach der vorliegenden Begrifflichkeit ein Polymer verstanden, dass eine Sulfongruppe in der Haupt- oder Nebenkette des Polymeren aufweist. Der Begriff Polysulfon (PSU) wird im Rahmen dieser Anmeldung als generischer Begriff für alle Polymere verstanden, die Sulfongruppen enthalten. Typische Vertreter von Materialien auf Polysufonbasis sind Polysulfon (PSU), Polyethersulfon (PES), Polyphenylsulfon und Copolymere enthaltend Sulfongruppen. Weitere Vertreter von Polysulfonpolymeren sind im Stand der Technik bekannt und im Sinne der Erfindung für die Herstellung von Blutbehandlungsmembranen geeignet, hier aber nicht weiter aufgelistet. Polysulfonmaterialien haben sich in der Herstellung von Blutbehandlungsmembranen gegenüber anderen Materialien als überlegen erwiesen, da sie dampfsterilisierbar sind und gute Eigenschaften hinsichtlich der Hämokompatibilität aufweisen.

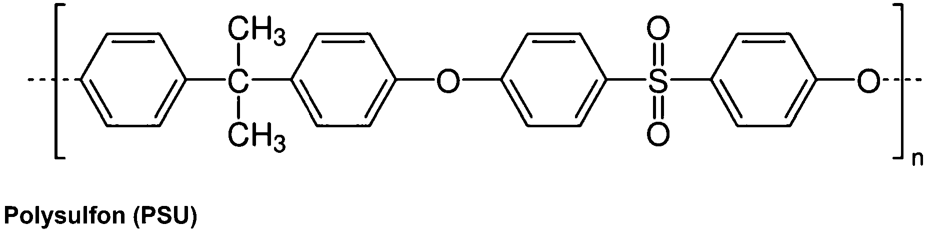

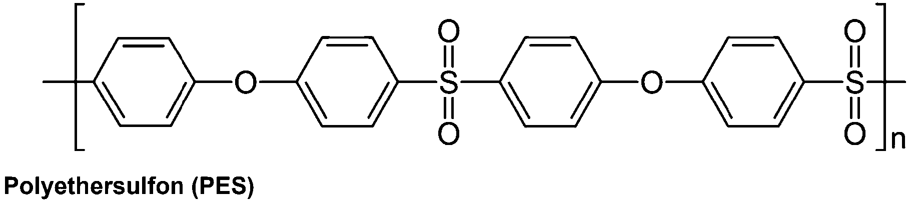

Unter einem Polymer auf Vinylpyrrolidonbasis wird ein Polymer verstanden, dass unter Verwendung des Monomeren Vinylpyrrolidon oder Derivaten davon hergestellt ist. Insbesondere eignet sich Polvinylpyrrolidon (PVP) im Rahmen der vorliegenden Erfindung für die Herstellung erfindungsgemäßer Hohlfasermembranen. PVP ist ein wasserlösliches Polymer, das als Hilfsmittel in der Produktion von Hohlfasermembranen auf Polysulfonbasis verwendet wird. Zudem bewirkt PVP eine Verbesserung der Hämokompatibilität von Hohlfasermembranen aus Polysulfon, da es das hydrophobe Polysulfonmaterial hydrophilisiert und dadurch besser für Blut benetzbar macht.

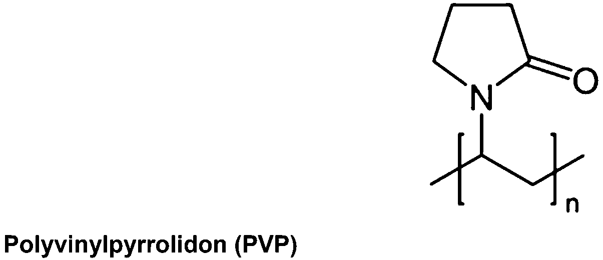

Unter Hämokompatibilität wird dabei die Verträglichkeit zu Humanblut verstanden, insbesondere wird darunter verstanden, dass Blut in Kontakt mit Polysulfonmaterialien keine negativen Reaktionen eingeht, die für den Patienten im Rahmen einer Blutbehandlungstherapie gesundheitsschädlich sein können. Beispielhaft können hierunter Blutgerinnungsphänomene oder die Neigung zur Blutzellschädigung (Zytotoxizität) verstanden werden. Die Verwendung von PSU/ PVP Polymeren haben sich gegenüber anderen Blutkontaktmaterialien in Hohlfasermembranen hinsichtlich ihrer Blutkomaptibilität als überlegen erwiesen.

Insbesondere sind Materialien von Hohlfasermembranen auf Polysulfon- und Polyvinylpyrrolidonbasis durch ihren Zetapotentialwert charakterisiert. Das Zetapotential ist ein Maß für die elektrische Ladungen, die an Oberflächen von Substraten vorliegen können. Insbesondere wurde an Blutbehandlungsmembranen diese Oberflächenladung in Zusammenhang mit schädlichen Reaktionen gebracht. Membranen auf Polysulfon und Polyvinylpyrrolidonbasis weisen abhängig von der Herstellmethode der Hohlfasermembran unterschiedliche Zetapotentialwerte auf.

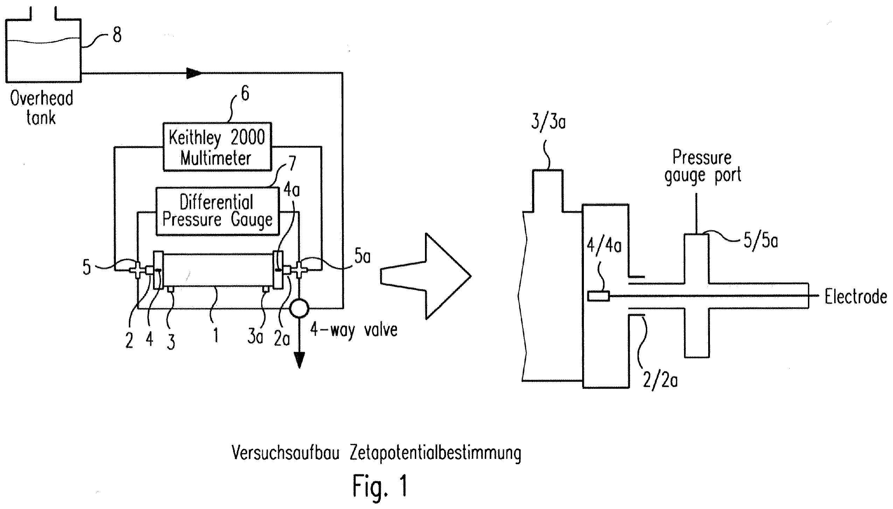

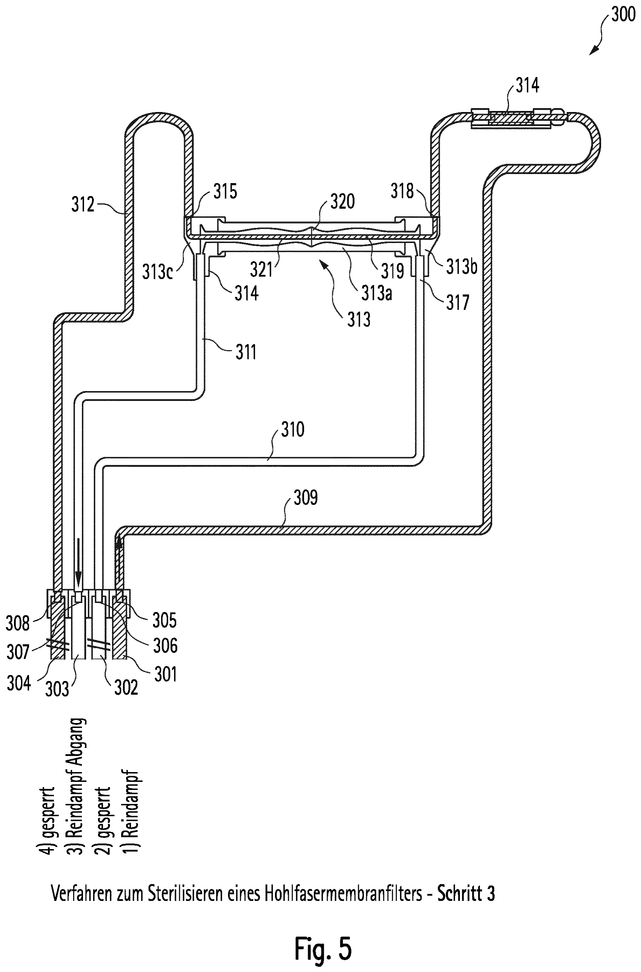

Die Porosität einer Membran gibt den Anteil des Porenvolumens eines Membranmaterials an. Bei Hohlfasermembranen wird hierbei nur der Anteil des Porenvolumens an der Membranwand in Betracht gezogen. Das Lumen einer Hohlfasermembran wird bei der Berechnung der Porosität nicht berücksichtigt. Die Porosität stellt ein Maß für die Durchlässigkeit einer Hohlfasermembran für Flüssigkeiten dar und ist damit auch ein Maß für die Abtrennleistung der Membran gegenüber Molekülen bestimmter Größe. Insbesondere wird im Zusammenhang mit dem Siebkoeffizienten eines Moleküls mit bestimmtem Molekulargewicht die Porosität als ein Maß für die Abtrennleistung der Membran für dieses Molekül angesehen. Im vorliegenden Fall wurde gefunden, dass eine Hohlfasermembran mit einem Siebkoeffizienten von 0,42 bis 0,75 für ein Dextran Molekül mit dem Molekulargewicht von 10000 g/mol, im Zusammenhang mit einer Porosität von 77,5% bis 82% von der Hohlfasermembran das im mittleren Molekulargewichtsbereich von Plasmaproteinen liegt, charakteristisch für eine hohe Permeabilität bzw. Clearance im Mittelmolekulargewichtsbereich und einer hohen Trennschärfe gegenüber hochmolekularen Plasmaproteinen, insbesondere Albumin, einer Hohlfasermembran ist. Bevorzugt ist eine Hohlfasermembran, die dadurch gekennzeichnet ist, dass sie im Wesentlichen keine Makrovoids oder dendritische Hohlräume aufweist. Dendritische Hohlräume sind als Makrovoids mit fingerartiger, länglicher Ausdehnung aufzufassen. Makrovoids sind in der angegebenen Literatur ("Mulder") beschrieben. Beispiele für die Ausprägung von dendritischen Hohlräumen finden sich zudem in der WO2004/056460 A1 Fig. 1, WO2013/034611 A1 Fig. 1, 2 und 3 oder WO2015/056460 A1 Fig. 5. Membranen ohne dendritische Hohlräume oder Makrovoids weisen eine höhere mechanische Stabilität auf. Weiter bevozugt ist eine Membran, die dadurch gekennzeichnet ist, dass sie eine Wandstärke von 35 µm oder weniger und im Wesentlichen keine Makrovoids oder dendritischen Hohlräume aufweist. Bei derartig niedrigen Wandstärken ist es besonders wichtig, eine gute mechanische Stabilität zu gewährleisten.

Der Siebkoeffizient gibt an, welcher Anteil von einer betrachteten Substanz bei einem Filtrationsprozess in der Lage ist, durch die Membranwand zu permeieren. Insbesondere gilt für eine Hohlfasermembran mit niedrigem Siebkoeffizient für hochmolekulare Moleküle, dass diese bei einer Filtration weitgehend von der Membranwand zurückgehalten werden und nur ein geringer Anteil durch die Poren der Hohlfasermembran permeieren kann. Man ist bestrebt in der Herstellung von Hohlfasermembranen für die Blutbehandlung eine Porenstruktur der Hohlfasermembran zu erschaffen, die eine hohe Rückhaltung von hochmolekularen Plasmaproteinen, wie z.B. Albumin ermöglicht und durch einen entsprechend kleinen Siebkoeffizienten für Albumin von 0,01 bevorzugt 0,005, besonders bevorzugt 0,001 charakterisiert ist. Dagegen setzt ein hoher Siebkoeffizient für Moleküle mit niedrigem Molekulargewichtvoraus, dass nahezu alle dieser Moleküle in der Lage sind, die Membranwand der Hohlfasermembran durch die Porenstruktur zu permeieren.

Die Porengrößenverteilung einer erfindungsgemäßen Membran ist dabei so strukturiert, dass sich der Siebkoeffizient für ein Dextran mit einem Molekulargewicht von 10000 g/mol, wie beschrieben von 0,42 bis 0,75 ergibt und der Siebkoeffizient für Albumin einen Wert, wie beschrieben von weniger als 0,1 annimmt.

In einer weiteren Ausführung gemäß des ersten Aspekts der Erfindung ist die erfindungsgemäße Hohlfasermembran durch einen Zetapotentialwert von -3mV bis -10mV gekennzeichnet. Insbesondere zeigte sich, dass schädliche Reaktionen mit Blutzellen innerhalb dieses Wertebereichs nur im geringen Maß auftreten.

In einer weiteren Ausführung des ersten Aspekts der Erfindung zeigte sich, dass die Trennschärfe der Hohlfasermembran verbessert werden konnte, wenn die Hohlfasermembran eine Porosität von 78% bis 81%, insbesondere eine Porosität für eine von 79% bis 80,5% aufweist.

In einer weiteren Ausführung gemäß des ersten Aspekts der Erfindung zeigte sich, dass die Trennschärfe der Hohlfasermembran weiter verbessert werden konnte, wenn die Hohlfasermembran einen Siebkoeffizienten für ein Dextranmolekül mit dem Molekulargewicht von 10000 g/mol von 0,45 bis 0,75, bevorzugt 0,55 bis 0,7, insbesondere einen Siebkoeffizienten von 0,6 bis 0,7 aufweist.

In einer weiteren Ausführung des ersten Aspekts der Erfindung zeigte sich, dass die Trennschärfe der Hohlfasermembranen verbessert werden konnte, wenn die Hohlfasermembran einen Siebkoeffizienten für Albumin von kleiner als 0,005 aufweist, insbesondere kleiner als 0,001 aufweist.

In einer weiteren Ausführungsform des ersten Aspekts der Erfindung zeigte sich, dass die Blutkompatibilität der Hohlfasermembran verbessert werden konnte, wenn die Hohlfasermembran ein Zeta-Potential von -4mV bis -8mV, insbesondere ein Zeta-Potential von -6mV bis -8mV aufweist.

In einer weiteren Ausführungsform des ersten Aspekts der Erfindung zeigte sich, dass die Blutkompatibilität der Hohlfasermembran verbessert werden konnte, wenn die Hohlfasermembran einen PVP Gehalt von 2,5% bis 5% aufweist.

In einer weiteren Ausführung des ersten Aspekts der Erfindung zeigte sich, dass die erfindungsgemäße Membran ein Maximum einer nominellen Porengrößenverteilung im Bereich von 22 bis 26 Å (Å= Angström= 100 ppm) aufweist. Insbesondere zeigte sich, dass mit einem Maximum der nominellen Porengrößenverteilung in diesem Molekulargewichtsbereich eine erwünschte hohe Abtrennung der mittelmolekularen Plasmaproteine erreicht werden kann. Die Porengrößenverteilung gibt dabei an, wie wahrscheinlich es ist, eine Pore mit einer bestimmten Porengröße unter allen vorhandenen Poren der Hohlfasermembran anzutreffen. Das Maximum der nominellen Porengrößenverteilung sagt damit aus, dass eine bestimmte Porengröße am häufigsten unter allen Poren aufzufinden ist. Es hat sich weiterhin im vorliegenden Fall herausgestellt, dass eine Regulierung der nominellen Porengrößenverteilung in der Herstellung einer Hohlfasermembran, so, dass das Maximum der nominellen Porengrößenverteilung im Bereich von 22 bis 26 Å, bevorzugt von 23 bis 26 Å, liegt, und der Siebkoeffizient für Albumin im Bereich bei kleiner 0,01 liegt, eine Membran mit verbesserter Trennschärfe hergestellt werden kann.

In einem zweiten Aspekt betrifft die Erfindung einen Hohlfasermembranfilter. Der Hohlfasermembranfilter besteht aus einem zylindrischen Gehäuse, das eine Vielzahl von Hohlfasermembranen enthält. Insbesondere können die Hohlfasermembranen nach einer der Ausführungen gemäß des ersten Aspekts der Erfindung ausgearbeitet sein. Die Hohlfasermembranen sind in dem Hohlfasermembranfilter an den Enden mit einer Vergussmasse so abgedichtet, dass ein erster Raum den inneren Raum der Hohlfasermembranen umfasst, und ein zweiter Raum den Raum zwischen den Hohlfasermembranen umfasst. Der Hohlfasermembranfilter umfasst weiter einen ersten Fluidzugang zur Zuleitung von Fluiden, insbesondere Flüssigkeiten oder Gasen in das Innere der Hohlfasermembranen und einen zweiten Fluidzugang zur Ableitung von Flüssigkeiten oder Gasen aus dem Inneren der Hohlfasermembranen. Der Hohlfasermembranfilter ist dadurch gekennzeichnet, dass die Hohlfasermembranen in verschiedenen Bereichen, insbesondere in einem Querschnitt des Hohlfasermembranfilters eine gleichmäßig verteilte Permeationseigenschaft, insbesondere einen einheitlichen Ultrafiltrationskoeffizienten aufweisen. Die Einheitlichkeit der Permeationseigenschaft der Hohlfasermembranen in den verschiedenen Bereichen des Hohlfasermembranfilters bemisst sich dadurch, dass die Hohlfasermembranen, die sich in unterschiedlichen Bereichen einen Ultrafiltrationskoeffizienten aufweisen, der nicht mehr als 20 % voneinander differiert.

Der einheitliche Ultrafiltrationskoeffizient der Hohlfasermembranen im Hohlfasermembranfilter ist darauf zurückzuführen, dass im Herstellverfahren ein Verfahrensschritt angewendet wird, bei dem ein transmembraner Übergang eines Fluids, insbesondere Wasserdampf oder Wasser, angewendet wird. Unter Wasserdampf wird im Sinne der vorliegenden Anmeldung die Bezeichnung für Wasser im gasförmigen Aggregatzustand verstanden. Im Sinne dieser Anmeldung ist unter Wasserdampf auch eine Form von gasförmigen Wasser zu verstehen, die von sogenannten sichtbaren Dampfschwaden, d.h. nebelförmiger, durch in der Luft verteilter Wassertröpfchen, begleitet wird. Zu dem Begriff Wasserdampf, wie er in der vorliegenden Anmeldung verwendet wird zählen damit auch andere Unterbezeichnungen von Wasserdampf, wie z.B. Heißdampf, Nassdampf, Sattdampf, gesättigter Dampf, überhitzter Dampf, überkritischer Dampf.

Der transmembrane Übergang des Fluids kann von dem ersten Raum, der das Innere der Hohlfasermembranen umfasst, durch die Membranwand in den zweiten Raum, der den Zwischenraum zwischen den Hohlfasermembranen umfasst, erfolgen. In einer alternativen Ausführung kann der transmembrane Übergang von dem zweiten Raum, der den Zwischenraum zwischen den Hohlfasermembranen umfasst, durch die Membranwand in den ersten Raum der das Innere der Hohlfasermembranen umfasst erfolgen. Es wird vermutet, dass der transmembrane Übergang eine Freispülung der Poren von PVP bewirken kann, so dass eine eventuell im Herstellverfahren aufgetretenen Verengung oder Blockierung der Poren der Hohlfasermembran durch abgelagertes PVP aufgehoben werden kann. Im Weiteren wird vermutet, dass durch den transmembranen Wasser- und/oder Wasserdampfübergang auch eine Zusammenlagerung der Hohlfasermembranen untereinander aufgehoben wird. Durch den transmembranen Übergang des Fluids, insbesondere des Wasserdampfs oder des Wassers, vom Inneren der Hohlfasermembranen auf die Außenseite der Hohlfasermembranen werden solche Zusammenlagerungen durch das anströmende Fluid aus dem Faserinneren gelöst. Dadurch wird insgesamt eine Auflockerung der Hohlfasermembranen innerhalb des Hohlfasermembranbündels beobachtet. Die Anströmung der Hohlfasermembranen durch das Fluid von außen und Überleiten durch die Membranwand in das Innere der Membran verursacht ebenfalls eine Auflösung von aneinanderhaftenden Hohlfasermembranen. Weiterhin wurde beobachtet, dass der Ultrafiltrationskoeffizient, gemessen am gesamten Hohlfasermembranfilter, steigt.

Ein Hohlfasermembranfilter im Sinn des zweiten Aspekts der Erfindung kann 50 bis 20000 Hohlfasermembranen umfassen, die in einer Packungsdichte von 50 bis 70% in dem Gehäuse des Hohlfasermembranfilters angeordnet sind. Als Packungsdichte ist dabei die Raumausfüllung von Hohlfasermembranen in einem Hohlfasermembranbündel zu bezeichnen, die in das Gehäuse eingebracht wurden. Die Packungsdichte von Hohlfasermembranen ist die Summe der Querschnittsflächen der einzelnen Hohlfasermembranen dividiert durch die Gesamtquerschnittsfläche, die alle Hohlfasermembranquerschnittsflächen in einer Anordnung umgrenzt. In der Regel ist dies der Gehäusequerschnitt. Bei Hohlfasermembranen und Gehäusegeometrien mit kreisrunden Querschnitt berechnet sich die Packungsdichte nach folgender Formel: ${δ}_{\left(Packungsdichte\right)} = n ⋅ \frac{{d}_{\left(Faser\right)}^{2}}{{d}_{\left(Gehäuse\right)}^{2}}$
- d_{(Faser)}: ist der mittlere Außendurchmesser der unbelasteten Hohlfasermembran
- d_{(Filter)}: ist der Innendurchmesser des Gehäuses
- n:: ist die Anzahl der Hohlfasermembranen im Gehäuse

Der Begriff unbelastete Hohlfasermembran bezieht sich auf eine einzelne freie Hohlfasermembran. Im Gehäuse können unter Kompressionswirkung die Hohlfasermembranen deformiert werden, d.h. unter Belastung einen deformierten Querschnitt annehmen. Zur Berechnung der Packungsdichte wird aber stets vom Durchmesser der unbelasteten Hohlfasermembran ausgegangen.

Die Auflockerung des Hohlfasermembranfilters durch den transmembranen Übergang des Fluids im Herstellverfahrens des Hohlfasermembranfilters ist dabei bei dicht gepackten, also bei Hohlfasermembranfiltern mit hoher Packungsdichte effektiver als bei Hohlfasermembranfiltern mit niedriger Packungsdichte. Insbesondere ist die Auflockerung der Hohlfasermembranen an Hohlfasermembranfiltern mit einer Packungsdicht der Hohlfasermembranen von 50 bis 70 %, bevorzugt 55 bis 65%, weiter bevorzugt 55 bis 65% als besonders effektiv anzusehen.

Insbesondere kann der transmembrane Übertritt durch das Fluid, insbesondere durch Wasserdampf oder Wasser, im Rahmen eines Hitzesterilisationsverfahrens durchgeführt werden, oder selbst Teil eines Hitzesterilisationsschritts sein. Im letzteren Fall ist vorgesehen, Wasserdampf bei einer Temperatur von 121 bis 140°C zu verwenden, so dass durch den transmembranen Wasserdampfübertritt auch eine keimabtötende Sterilisation erfolgt.

In einem dritten Aspekt betrifft die Erfindung ein Verfahren zur Herstellung von Hohlfasermembranbündeln zur Verwendung in einem Hohlfasermembranfilter, umfassend eine Vielzahl von Hohlfasermembranen. Insbesondere kann es sich bei dem Hohlfasermembranfilter um einen Hohlfasermembranfilter nach einer Ausführung des zweiten Aspekts der Erfindung handeln, weiter insbesondere können die Hohlfasermembranen nach einer Ausführung des ersten Aspekts der Erfindung ausgearbeitet sein. Das Herstellverfahren umfasst ein Spinnenverfahren von Hohlfasermembranen auf Polysulfon- und Polyvinylpyrrolidonbasis, insbesondere erfolgt das Spinnverfahren nach einem Dry-Wet Spinnverfahren. Das Herstellverfahren weißt die Verfahrensschritte auf:
- Bereitstellen einer Spinnlösung umfassend ein Material auf Polysulfonbasis, insbesondere Polysulfon, ein Polymer auf Basis von Vinylpyrrolidon, insbesondere Polyvinylpyrrolidon, einem aprotischen Lösungsmittel, insbesondere Dimethylacetamid,
- Bereitstellen einer Koagulationsflüssigkeit, umfassend Wasser und ein aprotisches Lösungsmittel, insbesondere Dimethylacetamid,
- Koextrusion der Spinnlösung und der Koagulationsflüssigkeit durch eine konzentrische Ringspaltdüse zu einem hohlen Spinnfaden, wobei der Hohlraum des Spinnfadens mit koextrudierter Koagulationsflüssigkeit befüllt ist,
- Durchleiten des Spinnfadens durch einen Fällspalt,
- Einleiten des Spinnfadens in ein Fällbad umfassend im Wesentlichen Wasser, um eine Hohlfasermembran zu erhalten,
- Durchleiten der Hohlfasermembranen durch wenigstens ein Spülbad und Trocknen der erhaltenen Hohlfasermembran,
- Anordnen der erhaltenen Hohlfasermembran zu einem Hohlfasermembranbündel,
- Behandeln des Hohlfasermembranbündels mit Wasserdampf.
Das Verfahren ist weiter dadurch gekennzeichnet, dass die Behandlung mit Wasserdampf wenigstens einen Schritt umfasst, bei dem Wasserdampf in das Innere der Fasern geleitet wird und durch Druckbeaufschlagung durch die Membranwand auf die Außenseite der Fasern permeiert wird.

Nach der Anordnung der Hohlfasermembranen zu einem Hohlfasermembranbündel und vor dem Behandeln des Hohlfasermembranbündels mit Wasserdampf kann das Hohlfasermembranbündel in ein Gehäuse eines Hohlfasermembranfilters eingebracht werden und mit einem aushärtbaren Harz an den Enden des Hohlfasermembranbündels nach Methoden, die im Stand der Technik bekannt sind, vergossen werden.

Das in das Gehäuse eingegossene Hohlfasermembranbündel kann zu einem Hohlfasermembranfilter weiterverarbeitet werden, so dass zwei Strömungsräume für Fluide entstehen, wobei ein erster Raum das Innere der Hohlfasermembranen umfasst und ein zweiter Raum den Zwischenraum zwischen den Fasern umfasst, und wobei der Hohlfasermembranfilter mindestens einen Zugang für Fluide zum ersten Raum des Hohlfasermembranfilters und mindestens einen Zugang für Fluide zum zweiten Raum des Hohlfasermembranfilters aufweist. Der Dampfbehandlungsschritt des Hohlfasermembranbündels kann anschließend im Inneren des Hohlfasermembranfilters durchgeführt werden, indem durch den ersten Zugang für Fluide, in den ersten Raum des Hohlfasermembranfilters der das Innere der Hohlfasermembranen umfasst, Wasserdampf eingeleitet wird und durch Druckbeaufschlagung über die Membranwand in den zweiten Raum des Hohlfasermembranfilters, der den Zwischenraum zwischen den Hohlfasermembranen umfasst, übergeleitet wird und über den zweiten Zugang am Hohlfasermembranfilter aus dem zweiten Raum ausgeleitet wird.

Der Behandlungsschritt mit Wasserdampf kann im Rahmen eines Spülverfahrens oder im Rahmen einer Hitzesterilisation durchgeführt werden, insbesondere stellt der Behandlungsschritt mit Wasserdampf selbst einen Spülschritt dar, wenn die Hohlfasermembranen als Hohlfasermembranbündel in einen Hohlfasermembranfilter eingearbeitet sind.

Die Herstellung eines Hohlfasermembranfilters nach dem vorab genannten Verfahren ermöglicht es, Hohlfasermembranfilter anzufertigen, deren Poren der Hohlfasermembranen frei von PVP Verblockungen oder Verengungen sind und bei dem die einzelnen Hohlfasermembranen sich nicht zusammenlagern. Dies hat zur Folge, dass die Clearance der Hohlfasermembranen in einem nach dem erfindungsgemäßen Verfahren hergestellten Hohlfasermembranfilter steigt, da effektiv eine größere Membranoberfläche für den transmembranen Stoffaustausch durch die Freilegung der einzelnen Fasern und die Freilegung der Poren von abgelagertem PVP bereitgestellt wird.

Weiterhin gewährleistet das Herstellverfahren eine hervorragende Biokompatibilität der Hohlfasermembran, wenn der Behandlungsschritt mit Wasserdampf im Rahmen einer Hitzesterilisation durchgeführt wird. In diesem Fall werden zerstörte Zellfragmente und Endotoxine, die durch die Sterilisationsbedingungen entstehen, von der Membranoberfläche ausgespült. Es ist daher in einer bevorzugten Ausführungsform vorgesehen das Hohlfasermembranbündel vor dem Behandlungsschritt mit Wasserdampf zu einem Filter weiterzuverarbeiten und die Wasserdampfbehandlung am Hohlfasermembranfilter im Rahmen eines Sterilisationsschrittes durchzuführen.

In einer weiteren Ausführung des dritten Aspekts der Erfindung wird für den Spinnprozess der Hohlfasermembranen eine Spinnlösung verwendet, die einen Anteil von 14 bis 18% eines Polymeren auf Basis von Polysulfon, vorzugsweise Polysulfon und einen Anteil eines Polymeren auf Basis von Vinylpyrrolidon, vorzugsweise Polyvinylpyrrolidon von 3 bis 6% umfasst. Weitere Anteile der Spinnlösung werden durch ein polar aprotisches Lösungsmittel gebildet, bevorzugt Dimethylacetamid (DMAC).

In einer weiteren Ausführung des dritten Aspekts der Erfindung ist das erfindungsgemäße Verfahren zur Herstellung eines Hohlfasermembranbündels dadurch gekennzeichnet, dass die Koagulationsflüssigkeit 25% bis 40 % eines polar aprotischen Lösungsmittels, insbesondere Dimethylacetamid, insbesondere 25% bis 40% DMAC und 60 bis 75% Wasser aufweist.

In einer weiteren Ausführung des dritten Aspekts der Erfindung ist das erfindungsgemäße Verfahren zur Herstellung eines Hohlfasermembranbündels dadurch gekennzeichnet, dass das Fällbad im Spinnprozess auf 75°C bis 85°C temperiert ist. Diese Fällbadtemperatur trägt zu einem hohen Ultrafiltrationskoeffizienten und einem hohen Siebkoeffizienten für Moleküle im mittleren Molekulargewichtsbereich bei.

In einer weiteren Ausführung nach dem dritten Aspekt der Erfindung ist das erfindungsgemäße Verfahren zur Herstellung eines Hohlfasermembranbündels dadurch gekennzeichnet, dass die Hohlfasermembranen bei einer Temperatur von 75°C bis 90°C gewaschen werden. Für den Waschvorgang wird Waschflüssigkeit, bevorzugt Wasser, in den Hohlfasermembranfilter geleitet und der Filter im ersten Raum und im zweiten Raum mit Wasser durchspült. Durch diesen Vorgang werden Restpartikel und eluierbare Bestandteile der Hohlfasermembran und des Filtergehäuses aus dem Hohlfasermembranfilter ausgespült.

In einer weiteren Ausführungsform nach dem dritten Aspekt der Erfindung ist das erfindungsgemäße Verfahren zur Herstellung eines Hohlfasermembranbündels dadurch gekennzeichnet, dass die Hohlfasermembranen bei einer Temperatur von 100°C bis 150°C getrocknet werden

In einer weiteren Ausführungsform des dritten Aspekts der Erfindung ist das erfindungsgemäße Verfahren dadurch gekennzeichnet, dass die Wassserdampfbehandlung des Hohlfasermembranbündels bei einer Temperatur von größer 60 bis 140°C durchgeführt wird.

In einem vierten Aspekt betrifft die Erfindung ein Sterilisationsverfahren zum Sterilisieren eines Hohlfasermembranfilters. Demnach wird ein Hohlfasermembranfilter aufweisend eine Vielzahl von Hohlfasermembranen, die an den Enden so in dem Gehäuse des Hohlfasermembranfilters abgedichtet sind, dass ein erster Raum entsteht, der das Innere der Hohlfasermembranen umfasst und ein zweiter Raum entsteht, der einen Raum zwischen den Hohlfasermembranen umfasst, sterilisiert. Der Hohlfasermembranfilter weist weiterhin mindestens zwei Fluidzugänge auf, die mit dem ersten Raum verbunden sind und mindestens zwei Fluidzugänge, die mit dem zweiten Raum verbunden sind, wobei die Fluidzugänge vorbereitet sind, mit einer Sterilisationsvorrichtung verbunden zu werden, und wobei das Verfahren wenigstens die Schritte umfasst:
- Spülen des Hohlfasermembranfilters mit einem Fluid, insbesondere Wasser, wobei das Spülfluid durch den ersten und den zweiten Raum des Hohlfasermembranfilters über eine Auswahl der Fluidzugänge geleitet wird,
- Sterilisieren des Hohlfasermembranfilters mit einem sterilisierenden Fluid, insbesondere Wasser oder Wasserdampf, wobei das Sterilisierfluid durch den ersten und den zweiten Raum des Hohlfasermembranfilters über eine Auswahl der Fluidzugänge geleitet wird,
- Zuführen eines Fluids, insbesondere Wasser oder Wasserdampf in den ersten oder den zweiten Raum des Hohlfasermembranfilters über eine Auswahl der Fluidzugänge, und
- transmembranes Überleiten des Fluids, insbesondere Wasser oder Wasserdampf, über die Membranwand hinweg in den jeweils zweiten oder ersten Raum der Hohlfasermembranfilters.

In einer Ausführungsform des erfindungsgemäßen Verfahrens nach dem vierten Aspekt der Erfindung ist vorgesehen, einen Spülschritt mit sterilem Wasser oder Wasserdampf, beziehungsweise sterilisierendem Wasser oder Wasserdampf an dem in den Hohlfasermembranfilter eingebauten Hohlfasermembranen, durchzuführen. Sterilisierend meint in diesem Fall, dass die Spülschritte unter Hitze- und Druckbedingungen durchgeführt werden. Sterilisierende Bedingungen im Rahmen einer Hitzesterilisation von Hohlfasermembranfiltern liegen bei Temperaturen von oberhalb 105°C bis 150°C, bevorzugt 121°C bis 140°C, und einem Absolutdruck von 1,1 bar bis 10 bar, vorzugsweise 2 bar bis 4 bar.

In einer weiteren Ausführungsform des vierten Aspekts der Erfindung umfasst das erfindungsgemäße Verfahren einen Schritt bei dem ein Fluid, insbesondere Wasser oder Wasserdampf, in den ersten Raum eines Filtermoduls eingeleitet wird, durch einen erzeugten Druckunterschied über die Membranwand in den zweiten Raum transportiert wird und dort abgeleitet wird. Alternativ kann das Fluid, insbesondere Wasser oder Wasserdampf, auch in den zweiten Raum des Hohlfasermembranfilters über eine Auswahl der Fluidzugänge zugeleitet werden und über einen erzeugten Druckunterschied von dem zweiten Raum über die Membranwand hinweg in den ersten Raum des Hohlfasermembranfilters geleitet werden. Die Fluidzugänge des Hohlfasermembranfilters sind dabei mit einer Sterilisationsapparatur verbunden, die in der Lage ist, sterilisierende Fluide, insbesondere erhitztes Wasser und/oder Wasserdampf, zu dem Hohlfasermembranfilter zu fördern. Vorzugsweise wird das Fluid, insbesondere Wasser oder Wasserdampf über einen ersten Fluidzugang in den ersten Raum gefördert und ein weiterer Fluidzugang zum ersten Raum des Hohlfasermembranfilters, sofern vorhanden, wird gesperrt. Es kann aber auch über beide Fluidzugänge gleichzeitig Fluid, insbesondere Wasser oder Wasserdampf, in den Hohlfasermembranfilter gefördert werden. In beiden Fällen bewirkt ein Druckaufbau durch das Fördern des Fluids, insbesondere Wasser oder Wasserdampf, dass das Fluid, insbesondere Wasser oder Wasserdampf, die Membranwand passiert und in den zweiten Raum übertritt. In dem zweiten Raum kann das übergetretene Fluid, insbesondere Wasser oder Wasserdampf, über einen weiteren Fluidzugang abgeführt werden.

In einer weiteren Ausführungsform des vierten Aspekts der Erfindung hat sich gezeigt, dass der transmembrane Übergang des Fluids, insbesondere Wasser oder Wasserdampf, bevorzugt vor einem Sterilisationsvorgang stattfindet.

In einer weiteren Ausführungsform des vierten Aspekts der Erfindung erfolgt die Sterilisation des Hohlfasermembranfilter über die Zuleitung einer Sterilisierflüssigkeit durch zwei Fluidzugänge, die zur Zuleitung von Fluiden in den ersten Raum und den zweiten Raum des Hohlfasermembranfilters vorgesehen sind. Die Fluide werden über jeweils zwei weitere Fluidzugänge zur Ableitung von Fluiden aus dem ersten Raum und dem zweiten Raum des Hohlfasermembranfilters abgeleitet, so dass die beiden Räume und der Filter von der Sterilisierflüssigkeit durchspült werden. Als Sterilisierflüssigkeit dient vorzugsweise steriles Wasser, dass auf eine Temperatur von 105°C bis 140°C temperiert ist.

In einer weiteren Ausführungsform des vierten Aspekts der Erfindung kann vorgesehen sein, dass ein Spülvorgang mit einer Flüssigkeit, insbesondere mit sterilem Wasser, durchgeführt wird. Alternativ sind wässrige Mischungen als Spülflüssigkeit einsetzbar. Bevorzugt findet das Spülen bei erhöhter Temperatur statt. Die Spülflüssigkeit kann dabei bevorzugt Temperaturen von 50°C bis 120°C annehmen. Insbesondere werden Partikel und weitere eluierbare Substanzen bei erhöhter Temperatur besser durch den Spülvorgang abtransportiert. Sofern das Membranmaterial auch hydrophile Bestandteile aufweist ist eine zu hohe Temperatur im Spülvorgang unerwünscht, da ein zu starkes Verkleben des Membranmaterials einsetzen kann. Bevorzugt ist eine Spültemperatur von 60°C bis 98°C, besonders bevorzugt ist eine Temperatur von 70 bis 98°C.

Die Sterilisation mit Wasserdampf findet bei Temperaturen von 124°C ± 5°C statt. Anlagentechnisch kann eine vorgewählte Temperatur nicht immer exakt eingehalten werden. Es hat sich daher technisch als sinnvoll erwiesen, Temperaturen zwischen 105°C und 140°C zu wählen. Dabei sind Drücke bis zu 4 Bar vorzusehen. Bei einer Sterilisationstemperatur von 124°C kann innerhalb von 12 Minuten die geforderte Sterilität erreicht werden. Alternativ kann auch bei niedrigeren Temperaturen bei längerer Sterilisationsdauer sterilisiert werden, z.B. bei 121°C für 15 Minuten.

Der transmembrane Übertritt mit einem Fluid, z.B. Wasser oder Wasserdampf, erfolgt vorzugsweise bei erhöhten Temperaturen. Dabei wird bevorzugt Wasserdampf in steriler Form verwendet. Insbesondere können durch den transmembranen Wasserdampfübertritt bei Temperaturen von 50°C bis 98°C auch Partikel und eluierbare Substanzen aus der Membranwand und den inneren Porenoberflächen gespült werden, die nicht direkt auf der Membranoberfläche liegen.

Es hat sich für den gesamten Sterilisations- und Spülablauf als vorteilhaft erwiesen, wenn zwischen dem ersten Spülschritt mit einem Spülfluid, insbesondere Wasser oder Wasserdampf, ein Spülschritt mit Druckluft oder einem alternativen Druckgas erfolgt. Dabei werden beide Räume des Filtermoduls mit steriler Druckluft ausgeblasen, ohne dass ein Druckgradient über das Membranmaterial zwischen erstem und zweitem Raum hergestellt wird. Dabei verbleibt Flüssigkeit aus dem vorangegangenen Spülvorgang in den Poren. Dieser Zwischenschritt erleichtert den nachfolgenden transmembranen Spülvorgang. Diese weitere Ausführungsform des vierten Aspektes der Erfindung ist also dadurch gekennzeichnet, dass ein weiterer Spülvorgang mit Druckgas, insbesondere mit steriler Druckluft, erfolgt.

**Beschreibung der Erfindung anhand von Messmethoden, Figuren und Beispielen**

### Messmethode 1: Bestimmung der Porosität

Ein Hohlfasermembranbündel, welches zuvor 2 Stunden bei 105°C im Trockenschrank getrocknet wurde, bestehend aus gleichen Hohlfasermembranen, wird gewogen. Es werden die mittlere Länge der Fasern, der mittlere Innendurchmesser und der mittlere Außendurchmesser und die Anzahl der Fasern bestimmt. Die mittleren Dimensionen werden an mindestens 10 verschiedenen Fasern des Hohlfasermembranbündels ermittelt. Zur Bestimmung der Dimensionen wird bei konstanter Temperatur von 20°C gearbeitet. Aus den Dimensionen wird ein Volumen ermittelt, das durch die Membranwandungen der Hohlfasermembranen des Hohlfasermembranbündels eingenommen wird, indem angenommen wird, dass die Geometrie der Hohlfasermembranen einem Hohlzylinder entspricht. Aus dem ermittelten Volumen und dem gemessenen Gewicht kann die mittlere Dichte der in den Hohlfasermembranen vorliegenden Membranstruktur errechnet werden. Die Porosität in Prozent ergibt sich aus dem Verhältnis von ermittelter zu theoretischer Dichte der Hohlfasermembran bei vollständiger Kompaktheit des Polysulfon-Materials gemäß folgender Formel: $Porosität = \frac{gemessene Dichte der Faser}{Dichte des Kompakten Polysulfon} ⋅ 100$

### Messmethode 2: Bestimmung des Zetapotentials

Für die Bestimmung des Zetapotentials der untersuchten Hohlfasermembranen wird ein Hohlfasermembranfilter (Dialysator) mit 10752 Hohlfasermembranen, die einen Innendurchmesser von 185 µm und eine Wandstärke von 35 µm aufweisen, verwendet. Die für die Messung des Zetapotentials relevante Länge der Hohlfasermembranen beträgt 279 mm. Die Hohlfasermembranen sind an den Enden in dem Hohlfasermembranfilter so vergossen, dass ein erster Raum entsteht, der das Innere der Hohlfasermembranen umfasst und ein zweiter Raum entsteht, der den Raum zwischen den Hohlfasermembranen umfasst. Als Vergussmaterial wird Polyurethan der Fa. Elastogran (Polyol C6947 und Isocyanat 136-20) verwendet. Die Vergusshöhe an jedem Bündelende beträgt 22 mm. Es wird eine Apparatur gemäß der Fig. 1 zur Messung verwendet. Der Hohlfasermembranfilter (1) weist Fluidzugänge (2, 2a, 3, 3a) zu dem jeweils ersten und zweiten Raum des Hohlfasermembranfilters (1) auf. Die Fluidzugänge zum ersten Raum des Hohlfasermembranfilters (1) werden mit je einer Ag/AgCl- Elektrode (4, 4a) und einem Zugang für die Druckmessung (5, 5a) versehen. Die Fluidzugänge (3, 3a) zum zweiten Raum des Hohlfasermembranfilters (1) werden dicht verschlossen, so dass der zweite Raum des Hohlfasermembranfilters unbefüllt ist. Zwischen beiden Elektroden wird somit die Spannungsdifferenz ΔE_{z} (mV) mit Hilfe eines Spannungsmessgerätes (6) aufgezeichnet, zwischen den Zugängen für die Druckmessung (5, 5a) wird der Druckabfall ΔP (N/m²) mit Hilfe eines Druckmessgerätes (7) aufgezeichnet. Die Prüfflüssigkeit besteht aus einer 1 mmolaren Lösung von KCI in Wasser mit einem pH- Wert von 7.4 und wird in einem Reservoir (8) vorgelegt, dass ca. 1000 mm oberhalb des Filters platziert wird. Die pH-Wert-Einstellung erfolgt nach folgender Vorschrift: Zu 100 Litern der KCI-Lösung werden 50 mg K₂CO₃ gegeben. Bei geöffnetem Behälter wird solange gerührt, bis sich ein pH-Wert von 7,4 einstellt. Dann wird der Behälter dicht verschlossen. Die Messung wird bei einer Temperatur von 23°C +/- 2°C durchgeführt.

Für die Messung des Zetapotentials wird die Prüfflüssigkeit durch einen ersten Fluidzugang (2) in den ersten Raum des Hohlfasermembranfilters eingeströmt, der den inneren Raum der Hohlfasermembranen umfasst und wird durch einen zweiten Fluidzugang (2a) am Hohlfasermembranfilter, der mit dem inneren Raum der Hohlfasermembranen in Verbindung steht, wieder aus dem Dialysator ausgeleitet. Der Hohlfasermembranefilter wird in dieser Anordnung zunächst 10 min. mit der Prüfflüssigkeit gespült, bis sich ein stabiler Wert eingestellt hat, ggf. wird weitere 5 min. gespült. Die Druckdifferenz und die Spannungsdifferenz wird zeitgleich an der Druckmessvorrichtung bzw. dem Multimeter abgelesen und daraus das Zetapotential berechnet. Zur Erhöhung der Messgenauigkeit ist vorgesehen, dass nach der Messwertaufnahme die beiden 4-Wegeventile so umgeschaltet werden, dass sich ein umgekehrter Fluss der Prüfflüssigkeit durch den inneren Raum der Hohlfasermembranen ergibt. Der Messwert für das Zetapotential wird dann aus dem Mittelwert der Messungen in beiden Flussrichtungen gebildet.

Die Berechnung des Zetapotentials erfolgt nach folgender Gleichung: $ζ = \frac{η ∗ Λo ∗ d Ez}{εo ∗ εr ∗ d ΔP}$
mit ζ = Zetapotential (mV)
η = Lösungsviskosität (0,001 Ns/m² )
Λₒ = Leitfähigkeit der Lösung (A/(V*m))
εₒ = Permittivität des Vakuums (8,85 * 10⁻¹² A * s /(V * m)
εᵣ = relative Permittivität der Lösung ( 80 )
E_{Z} = Strömungspotential ( mV)
Δ_{P} = Druckdifferenz ( N /m² )

### Messmethode 3: Bestimmung des Dextran- Siebkoeffizienten

Die Messung des Dextran- Siebkoeffizienten einer Hohlfasermembran wird in Anlehnung an die DIN EN ISO 8637:2014 an einem fertig aufgebauten Hohlfasermembranfilter durchgeführt. Dazu wird ein Filter mit 10752 Hohlfasermembranen mit einem Innendurchmesser von 185 µm und einer Wandstärke von 35 µm verwendet. Die aktive Länge der Hohlfasermembran beträgt 235 mm. Unter einer aktiven Länge einer Hohlfasermembran ist die Länge der Hohlfasermembran ohne Verguss zu verstehen, die für die Bestimmung der Permeationseigenschaften, wie Siebkoeffizient, Clearance und Ultrafiltrationskoeffizient zur Verfügung steht. Der Innendurchmesser des Hohlfasermembranfilters beträgt in der Mitte 34 mm. Der Hohlfasermembranfilter weist ansonsten den gleichen Aufbau auf, wie er in "Messmethode 2" beschrieben ist. In Abweichung zur Norm wird als Prüfflüssigkeit eine wässrige Dextranlösung mit einer breiten Molekulargewichtsverteilung des gelösten Dextrans zwischen 1000 und 100000 Da oder eine Mischung aus mehreren Dextranen in diesem Molekulargewichtsbereich verwendet, so dass sich die angegebene Verteilung des Molekulargewichts ergibt. Die Dextranlösung wird durch die Fluidzugänge, durch den ersten Raum des Hohlfasermembranfilters, der das Innere der Hohlfasermembranen umfasst mit einem Fluss von 446,6 ml/min durchgeleitet. Im zweiten Raum des Hohlfasermembranfilters wird über die Fluidzugänge ein Fluss von 89,9 ml/min von reinem Wasser eingestellt. Nach 12 Minuten wird die Konzentration der Dextrane, abhängig vom jeweiligen Molekulargewicht am ersten und zweiten Fluidzugang des ersten Raums des Hohlfasermembranfilter über den gesamten Molekulargewichtsbereich mit Hilfe der Gelpermeationschromatographie ermittelt und daraus eine Siebkoeffizientenkurve über den gesamten Molekulargewichtsbereich bestimmt. Der Siebkoeffizient eines Dextranmoleküls mit einem bestimmten Molekulargewicht kann dann aus der Siebkoeffizientenkurve ermittelt werden.

### Messmethode 4: Bestimmung des Albumin Siebkoeffizienten

Der Albumin-Siebkoeffizient einer Hohlfasermembran wird an einem Filter wie in Messmethode 3 durchgeführt. Zur Messung wird ein Humanplasma gemäß der Norm DIN EN ISO 8637:2014 zur Bestimmung des Siebkoeffizienten verwendet. Es wird also der "Plasma- Siebkoeffizient" des Albumins bestimmt. Als Analysengerät findet das Modell Cobas Integra 400 plus der Fa. Roche Diagnostics GmbH, Mannheim Verwendung. Die Durchführung der Messung erfolgt mit Hilfe des Testes ALBT2 in der Applikation für Urin. Es wird ein Plasmafluss von 446,6 ml/ min. und ein Dialysatfluss (deionisiertes Wasser) von 89,9 ml / min. eingestellt.

### Messmethode 5: Bestimmung der Clearance für Natrium, Phosphat und Vitamin B12

Die Bestimmung der Clearance einer Hohlfasermembran wird anhand eines nach Messmethode 2 aufgebauten Hohlfasermembranfilters nach der DIN EN ISO 8637:2014 durchgeführt. Als Prüflösungen für den Blutbereich (Blutbereich entspricht dem ersten Raum des Hohlfasermembranfilters, umfassend das Innere der Hohlfasermembranen) werden gemäß 5.6.1.2 der Norm wässrige Lösungen von Natrium in einer Konzentration von 5 g/l NaCl und 0,05 g/l Vit B12 Vitamin B12 verwendet, für den Dialyseflüssigkeitsbereich (Dialysierflüssigkeitsbereich entsprcht dem zweiten Raum des Hohlfasermembranfilters, umfassen den Faserzwischenraum) wird hierbei destilliertes Wasser verwendet. Phosphat wird in einer Konzentration von 3 mmol/I in der Dialysierflüssigkeit verwendet, die Messung wird ebenso gegen Dialysierflüssigkeit auf der Dialysatseite durchgeführt. Für Phosphat wird folgende Dialysierflüssigkeit angesetzt: 34,63 I Wasser, 102,9 g NaHCO₃, 210,68 g NaCl, 2,61 g KCl, 5,15 g CaCl₂* 2 H₂O, 3,56 g MgCl₂ * 6H₂O, 6,31g CH₃COOH, 38,5 g Glukosemonohydrat. Phosphat wird photometrisch nach Reaktion mit Ammoniummolybdat in schwefelsaurer Lösung bestimmt, wobei das Gerät Cobas integra 400 plus der Fa. Roche Diagnostics GmbH, Mannheim, Deutschland, und der Test PHOS2 (Roche) Verwendung findet. Die Natriumkonzentration wird durch Leifähigkeitsmessungen ermittelt. Die Konzentration von Vitamin B12 wird photometrisch bestimmt. Für die Clearance-Versuche wird ein baugleicher Hohlfasermembranfilter herangezogen, der auch für die Messung nach Messmethode 2 verwendet wird. Im ersten Raum des Hohlfasermembranfilters, der das Innere der Hohlfasermembranen umfasst, wird für die im Rahmen dieser Anmeldung hergestellten Hohlfasermembranfilter ein Fluss von 300ml/ min, im zweiten Raum des Hohlfasermembranfilters wird ein Fluss von 500ml/min eingestellt.

### Messmethode 6: Bestimmung des lokalen Ultrafiltrationskoeffizienten

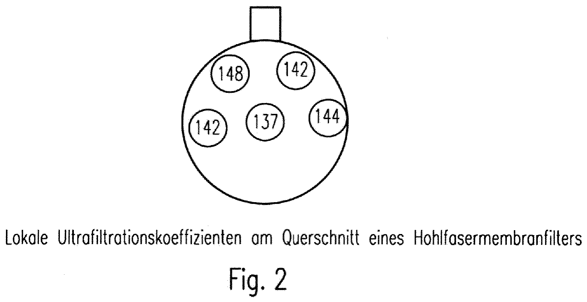

Für die Bestimmung des lokalen Ultrafiltrationskoeffizienten wird ein Hohlfasermembranfilter mit 10752 Hohlfasermembranen mit einem Innendurchmesser von 185 µm und einer Wandstärke von 35 µm verwendet, wie es bei "Messmethode 3" beschrieben ist. Die aktive Länge der Hohlfasermembran beträgt 235 mm. Unter aktiver Länge der Hohlfasermembran ist die Länge der Hohlfasermembran ohne Verguss zu verstehen, die für die Bestimmung der Permeationseigenschaften, wie Siebkoeffizient, Clearance und Ultrafiltrationskoeffizient, zur Verfügung steht. Der Innendurchmesser des Hohlfasermembranfilters beträgt in der Mitte 34 mm. Die Einlasskappe der Blutseite des Filters wird von dem Hohlfasermembranmodul entfernt und durch einen Einlass ersetzt, der eine Vorrichtung enthält, die den Fluss der Testflüssigkeit nur auf einen kreisförmigen Teil des Hohlfaserbündels mit einem Durchmesser von 1 cm lenkt. Dabei wird abweichend von der Norm DIN ISO 8637:2014 als Testflüssigkeit Wasser verwendet, es wird also der dem Fachmann bekannte "wässrige Ultrafiltrationskoeffizient" bestimmt. Diese Vorrichtung ist so ausgestaltet, dass das Ende dieser Vorrichtung ca. 3 mm in das obere Ende des Hohlfasermembranbündels eindringt und so zu einer Abdichtung der Vorrichtung gegenüber dem Hohlfasermembranbündel führt. Somit ist sichergestellt, dass nur ein lokaler kreisförmiger Flächenbereich mit dem Durchmesser 1 cm gemessen wird. Für die Messung weiterer Stellen wird entweder eine modifizierte Vorrichtung verwendet oder die Vorrichtung neu an der gewünschten Stelle positioniert. Eine schematische Darstellung der Bereiche am Querschnitt eines Hohlfasermembranfilters ist der Fig. 2 zu entnehmen. Bei der Einstellung der Flüsse der Prüfflüssigkeit wird darauf geachtet, dass die gleichen transmembranen Drücke (TMP) eingestellt werden wie bei der Messung des wässrigen Ultrafiltrationskoeffizienten in Anlehnung an die DIN ISO 8637:2014. Der höchste eingestellte TMP beträgt 600 mm Hg.

### Messmethode 7: Bestimmung des PVP Gehalts der Hohlfasermembran

Die Bestimmung des PVP-Gehaltes der Hohlfasermembran wird mit Hilfe der IR-Spektroskopie durchgeführt. Dazu wird die Probe zunächst 2 Stunden im Trockenschrank bei 105°C getrocknet. Dann wird 1 g der Faser in Dichlormethan aufgelöst. Zusätzlich werden Kalibierstandards unter Verwendung von getrocknetem PVP, welches ebenfalls in Dichlormethan aufgelöst wird, erstellt. Dabei wird ein Konzentrationsbereich von ca. 1% bis 10% PVP in der Hohlfaser abgedeckt. Die Lösungen werden jeweils in eine Flüssigküvette mit einer Schichtdicke von 0,2 mm verbracht. Zur Auswertung wird die Absorptionsbande der Carbonylfunktion herangezogen.

### Messmethode 8: Darstellung der nominellen Porengrößenverteilung und der nominellen mittleren Porengröße.

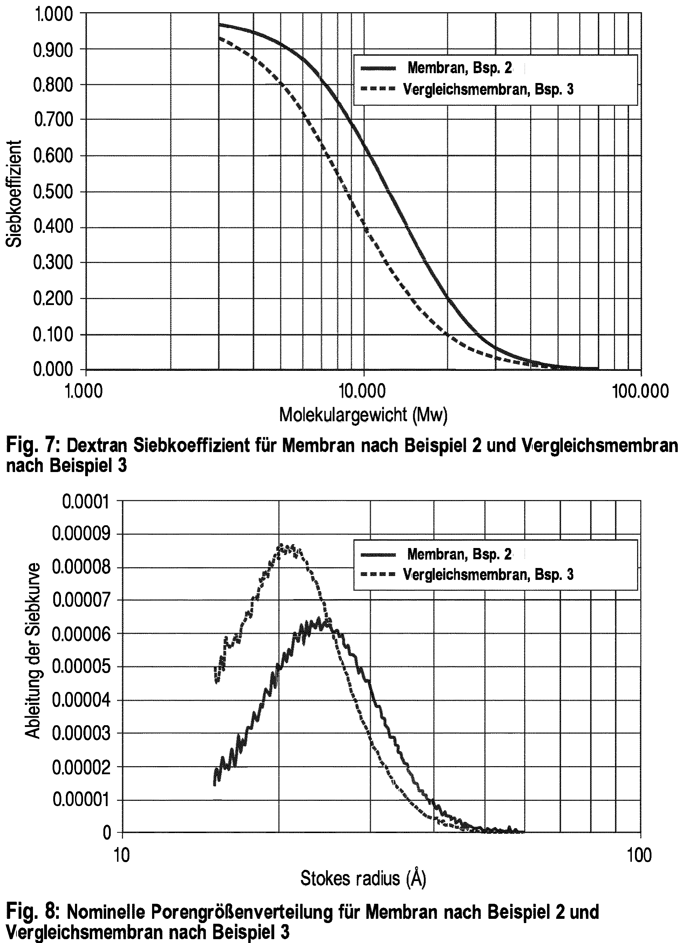

Ein Maß für die Porengrößenverteilung einer erfindungsgemäßen Membran kann ausgehend von der von der Siebkoeffizientenkurve, wie in Fig. 7 dargestellt abgeleitet werden. Hierzu wird die Siebkoeffizientenkurve, wie in der Messmethode 3 beschriebenen, für eine im Molekulargewicht breit verteilte Dextranprobe oder einer Mischung aus mehreren
Dextranproben erhalten. Die Siebkoeffizientenkurve liefert für jedes Molekulargewicht eine Durchgangswahrscheinlichkeit mit der entsprechende Dextranmoleküle die Membranwand passieren können. Das Molekulargewicht korreliert bei gegebener Temperatur und gegeben Lösungsmittel mit einer bestimmten Molekülgröße, die durch den Stokesradius beschrieben werden kann. Die Beziehung zwischen Stokes Radius und Molekulargewicht liefert die Gleichung nach J. Bandrup, E.H. Immergut ("Polymer Handbook" (1989) VII pp. 112-113, John Wiley): $Stokes Radius \left[Å\right] = 0 , 4456 ⋅ {M}^{0 , 43821}$ wobei M für das Molekulargewicht des Dextrans steht. Die Umrechnung von Molekulargewicht in Stokesradius wird für jeden Datenpunkt der Siebkoeffizientenkurve nach Fig. 7, z.B. mit Hilfe einer Computer Software, wie z.B. des Programms "Excel", durchgeführt. Eine entsprechende Darstellung analog zur Siebkoeffizientenkurve gibt eine Durchtrittswahrscheinlichkeit wieder, mit der Dextranmoleküle mit bestimmten Stokes Radius, d.h. mit bestimmter Molekülgröße, in der Lage sind, die Membranwand zu passieren. Gleichzeitig liefert diese Darstellung eine Aussage über die Struktur der Porengrößenverteilung, indem dadurch angegeben wird, wie groß die Wahrscheinlichkeit ist, eine Pore der Membran mit einer bestimmten Größe vorzufinden, so dass diese in der Lage ist ein Dextranmolekül mit vorbestimmter Molekülgröße, d.h. Stokesrqadius, passieren zu lassen.
Im Weiteren wird durch Umrechnung mit Hilfe des Softwareprogramms Excel die erste Ableitung in jedem Punkt der Kurve gebildet. Der daraus erhaltene Kurvenverlauf gibt eine Verteilungskurve wieder, die ein Maß für eine nominelle Porengrößenverteilung der untersuchten Membran darstellt. Ein entsprechender Kurvenverlauf ist in Fig. 8 für eine erfindungsgemäße Membran und eine Vergleichsmembran gezeigt. Das Maximum der Verteilungskurve gibt eine nominelle mittlere Porengröße der Membranen wieder.

### Beispiel 1: Verfahren zum Reinigen eines Hohlfasermembranfilters

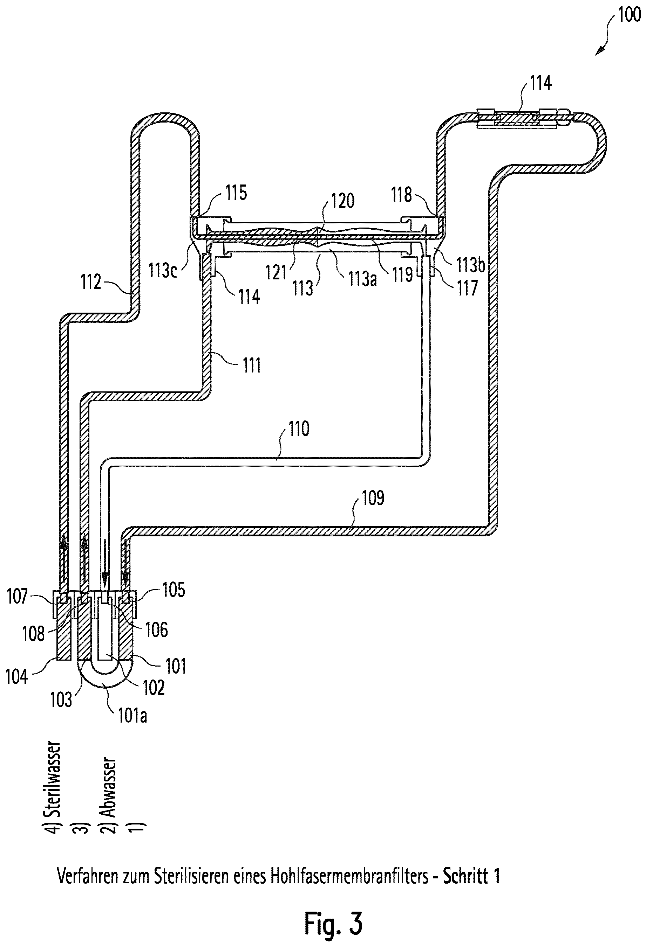

Fig. 3 zeigt in schematischer Darstellung einen ersten Schritt eines Reinigungsvorgangs eines Hohlfasermembranfilters, umfassend Spül- und Sterilisationsschritte, wie er in der Herstellung erfindungsgemäßer Hohlfasermembranen, beziehungsweise von Hohlfasermembranfiltern nach dem ersten, zweiten und dritten Aspekt der Erfindung verwendet wird. Fig. 3 zeigt einen Fluidzugang 118 zu einem ersten Raum 119 eines Hohlfasermembranfilters 113, der den Innern Raum der Hohlfasermembranen umfasst, der über eine Leitung 109 mit einem Ventil 105 mit einem Anschluss 101 in Fluidverbindung steht. Ein weiterer Fluidzugang 117 steht über Leitung 110, Ventil 106 mit Anschluss 102 in Fluidverbindung und bildet einen Zugang zu einem zweiten Raum 120 des Hohlfasermembranfilters 113, der einen Raum zwischen den Hohlfasermembranen umfasst. Ein weiterer Fluidzugang 114 steht über Leitung 111 mit Ventil 107 und dem Anschluss 103 in Fluidverbindung und bildet einen Zugang zu dem einen zweiten Raum 120 des Hohlfasermembranfilters. Fluidzugang 115 steht über Leitung 112 mit einem Ventil 108 und dem Anschluss 104 in Fluidverbindung. Weiterhin steht Anschluss 101 und 103 über das Verbindungsstück 101a in Fluidverbindung.

In einer beispielhaften Ausführung eines Spülvorgangs wird in dem gezeigten ersten Schritt über Anschluss 104 eine Spülflüssigkeit durch Leitung 112 zum Hohlfasermembranfilter 113 gefördert. Vorzugsweise handelt es sich bei der Spülflüssigkeit um temperiertes Sterilwasser, wobei Temperaturen von 50 bis 98°C eingehalten werden. Ventil 108 ist dabei auf Durchfluss geschaltet. Die Spülflüssigkeit strömt über den zweiten Fluidzugang 115 in den ersten Raum 119 des Hohlfasermembranfilters ein und verlässt diesen ersten Raum über den ersten Fluidzugang 118. Mit Hilfe der Anordnung werden sämtliche Hohlfasermembranen eines Hohlfasermembranbündels auf ihrer Innenseite durchspült.

Die Spülflüssigkeit durchläuft im Weiteren einen Blasendetektor 114, der in diesem Spülvorgang keine Funktion übernimmt, und Leitung 109 und wird über Anschluss 101 und Verbindungsstück 101a zu Leitung 111 gelenkt. Über den Fluidzugang 114 tritt die Spülflüssigkeit in den zweiten Raum 120 des Filtermoduls 113 ein und durchspült den zweiten Raum der im Zwischenraum zwischen den
Hohlfasermembranen gebildet wird. Über Fluidzugang 117 und Leitung 110 erfolgt ein Rücklauf der Spülflüssigkeit, die entweder verworfen wird oder durch eine Aufbereitung wieder für einen Spülvorgang zu Verfügung steht.

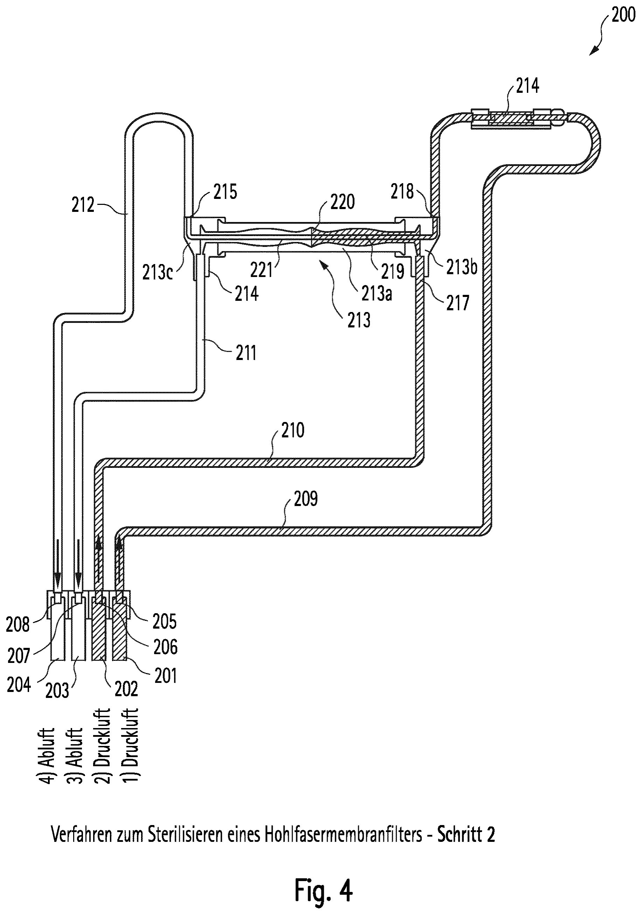

Fig. 4 zeigt in schematischer Darstellung einen zweiten Schritt eines Spül- und Sterilisationsvorgangs, wie er in der Herstellung erfindungsgemäßer Hohlfasermembranen, beziehungsweise von erfindungsgemäßen Hohlfasermembranfiltern nach dem ersten, zweiten und dritten Aspekt der Erfindung verwendet wird. Anhand Fig. 4 wird eine Druckluftspülung erläutert. Anschlüsse 201, 202, werden von einer Druckluftquelle versorgt, die sterile Luft liefert. Die Druckluft wird über die Leitungen 209 und 201 über die geöffneten Ventile 205 und 206 und durch nicht gezeigte Pumpenmittel zu dem Hohlfasermembranmodul 213 gefördert. Aus dem voran gegangenen Spülschritt gemäß dem Spülvorgang nach Fig. 3 sind der erste Raum 219 und der zweite Raum 220 zunächst noch mit Wasser gefüllt. Die Ventile 207 und 208 sind geöffnet und für eine Ableitung von Spülflüssigkeit vorbereitet. Mit einem Druck von 1,5 bis 2 bar wird die Druckluft durch das Filtermodul gefördert. Die Druckluft fördert dabei über die Fluidzugänge 218, 217 Restwasser jeweils aus dem ersten und dem zweiten Raum des Hohlfasermembranfilters weiter über die Fluidzugänge 215 und 214 in den rücklaufenden Teil der Flusswegschaltung. Über die Leitungen 212, 211 wird Restwasser und Druckluft abgeleitet. Der Spülvorgang wird für 2 bis 5 Minuten durchgeführt. Da in beiden Räumen 219 und 220 gleicher Druck vorherrscht wird nicht über die Membranwandung hinweg gespült. Als Folge bleiben die Poren der Membranwandung aus dem Spülvorgang wassergefüllt.

Fig. 5 zeigt in schematischer Darstellung einen dritten Schritt eines Spül- und Sterilisationsvorgangs, wie er in der Herstellung erfindungsgemäßer Hohlfasermembranen, beziehungsweise von erfindungsgemäßen Hohlfasermembranfiltern nach dem ersten, zweiten und dritten Aspekt der Erfindung verwendet wird. In Fig. 5 sind die Anschlüsse 302 und 304 dargestellt, die durch eine sperrende Ventilstellung der Ventile 306 und 308 den Ablauf von Spülfluiden sperrt. Wasserdampf
wird über Anschluss 301 in das Sterilisationssystem gefördert und über Leitung 309 zum Filtermodul 313 gefördert. Der Wasserdampf breitet sich im ersten Raum 319 des Hohlfasermembranfilters aus, eine Ableitung über den Fluidzugang 315 ist nicht möglich, da Anschluss 304 gesperrt ist. Eine Ausbreitung von Wasserdampf in Leitung 312 kann nur über Kompression des unter Druck stehenden Reindampfes oder Diffusion erfolgen.

Da im ersten Raum ein höherer Druck als im zweiten Raum besteht, erfolgt ein transmembraner Übergang des Reindampfes. Restwasser, das aus dem Spülprozess nach dem ersten Schritt der des Spül- und Sterilisationsvorgangs gemäß Fig. 3 in den Poren verblieben ist, wird abtransportiert und über den zweiten Raum 320 in Leitung 311 gefördert. Leitung 310 dient dabei aufgrund des gesperrten Anschlusses 302 nicht der Förderung von Fluiden. Aneinander liegende Hohlfasermembranen werden durch den transmembranen Spülvorgang weitestgehend voneinander gelöst. Wasserdampf wird dabei mit einem Druck von 1,3 bis 2 bar in das Filtermodul gefördert. Das Freispülen der Poren verhindert zusätzlich eine Adhäsion der Hohlfasern. Dieser Spülfortgang kann nach einigen Minuten abgeschlossen sein. Insbesondere wird der Spülvorgang für 2 bis 5 Minuten durchgeführt. Es werden Temperaturen von 50°C bis 98°C eingehalten, insbesondere auch um das Filtermodul für den nachfolgenden Sterilisationsvorgang thermisch zu konditionieren.

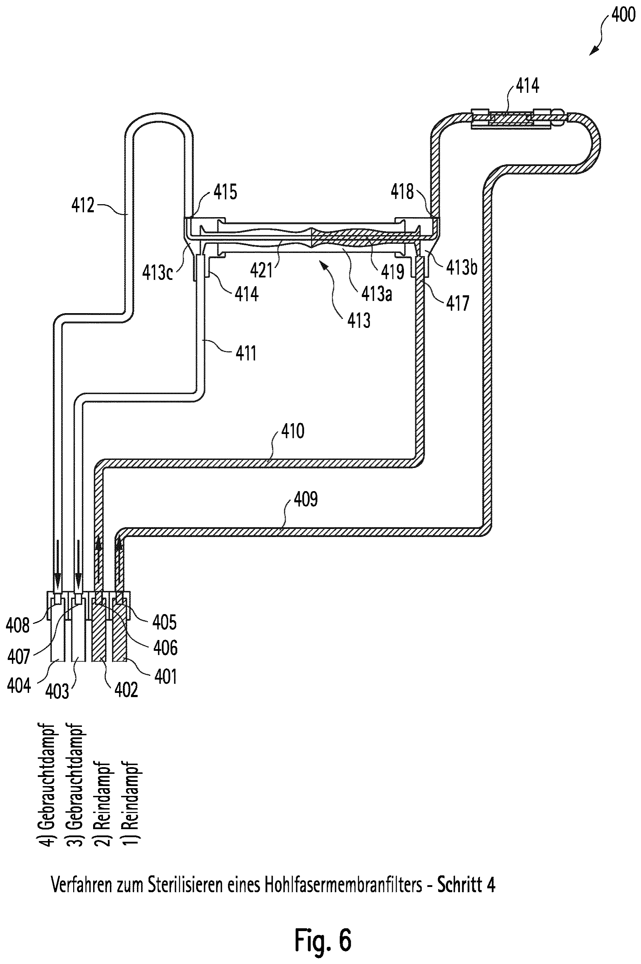

Fig. 6 zeigt in schematischer Darstellung einen vierten Schritt eines Spül- und Sterilisationsvorgangs, wie er in der Herstellung erfindungsgemäßer Hohlfasermembranen, beziehungsweise von erfindungsgemäßen Hohlfasermembranfiltern nach dem ersten, zweiten und dritten Aspekt der Erfindung verwendet wird. Gemäß dem vierten Schritt wird ein sterilisierendes Fluid wie z.B. Wasserdampf bei einer Temperatur von 124°C und einem Druck von 2 bar in den Hohlfasermembranfilter gefördert. Anschlüsse 401, 402, 403, 404 sind dabei durch die offenen Ventile 405 bis 408 durchströmbar. Über die Leitungen 409 und 410 wird der Reindampf in den Hohlfasermembranfilter gefördert und der erste Raum 419 und der zweite Raum 420 des Filtermoduls 413 durchspült. Der Rücklauf des Reindampfes erfolgt über die Leitungen 412 und 411 und die Fluidzugänge 415 und 414 und wird entweder verworfen oder durch Aufbereitung wiederverwendet. Die Dauer des Sterilisationsvorganges kann entsprechend der gewählten Sterilisationstemperatur 5 bis 30 Minuten betragen. Bei der bevorzugten Temperatur von 124°C ist eine Sterilisation nach 12 Minuten als vollständig anzusehen. Es können sich weitere Spülschritte anschließen um den Hohlfasermembranfilter in eine gereinigte und sterile Gebrauchsform zu bringen.

Für die weitere Qualitätsprüfung schließt sich ein aus dem Stand der Technik bekannter "bubble point" Test an. Dieser Test stellt einen Druckhaltetest dar, bei dem eine Seite einer Membran mit einem Gas mit einem höheren Druck beaufschlagt wird, als die gegenüberliegende Seite der Membran, die von einer Flüssigkeit angeströmt wird. Hierzu wird der zweite Raum 120, 220, 320, 420 der in den Fig. 3 bis Fig. 6 gezeigten Hohlfasermembranfilter mit steriler Druckluft gespült, wobei der erste Raum aus dem Spülvorgang flüssigkeitsgefüllt bleibt. Es wird durch das Sterilisationssystem im zweiten Raum ein höherer Druck angelegt, als im ersten Raum 119, 219, 319, 419. Da die Poren aus dem vorangehenden Spülschritt wassergefüllt sind, wird ein Übertritt des Druckgases vom ersten Raum in den zweiten Raum erst einsetzen, wenn der angelegte Druck die Oberflächenspannung des Wassers in den Poren überwindet. Die in den ersten Raum übertretenden Gasmengen können in den gezeigten Blasendetektoren 114, 214, 314, 414, analysiert werden und die Ergebnisse entsprechend bewertet werden. Über die Menge der detektierten Gasblasen korrelierend zu einem angewendeten Druck im zweiten Raum der Filtermodule 120 bis 420, können Rückschlüsse über die Qualität des Membranmaterials getroffen werden, und entschieden werden ob das Filtermodul spezifikationskonform ist.

Anschließend kann vorgesehen sein, dass der erste Raum ebenfalls mit steriler Druckluft gespült wird. Ein weiterer Spülschritt mit Reindampf kann in gegebenen Fällen dafür sorgen, dass verbliebene Wasser aus vorangehenden Spülprozessen zu entfernen. Im Anschluss kann ein Trocknungsprozess erfolgen bei dem das Filtermodul mit steriler Druckluft durchspült wird, bis ein angestrebter Trocknungsgrad erreicht ist

### Beispiel 2: Ausführungsbeispiel einer erfindungsgemäßen Hohlfasermembran

Eine Spinnlösung bestehend aus 16 Gewichtsteilen Polysulfon (P3500 der Fa. Solvay), 4,4 Gewichtsteilen Polyvinylpyrrolidon (K82-86 der Fa. Ashland) und 79,6 Gewichtsteilen DMAC werden unter Rühren, Erwärmen auf 60°C und Entgasen zu einer homogenen Spinnmasse verarbeitet. Die Spinnmasse wird durch eine Ringspaltdüse mit einem zentral geführten Fällmittel, bestehend aus 35% DMAC und 65% Wasser, zu einem Spinnfaden extrudiert. Das Fällmittel wird im Inneren des hohlen Spinnfadens geführt. die Temperatur der Ringspaltdüse beträgt 70°C. Der extrudierte Spinnfaden wird durch einen Fällraum geführt, dessen Atmosphäre eine relative Feuchtigkeit von 100 % aufweist. Die Höhe des Fällspaltes beträgt 200mm, es wird eine Verweilzeit im Fällspalt von 0,4 sec. eingestellt. Der Spinnfaden wird in Fällbad bestehend aus Wasser eingeleitet, das auf 80°C temperiert ist und zu einer Hohlfasermembran ausgefällt. Anschließend wird die Hohlfasermembran durch Spülbäder, die auf eine Temperatur von 75°C bis 90°C temperiert sind geleitet. Anschließend durchläuft die Hohlfasermembran einen Trocknungsprozess zwischen 100°C und 150°C. Die erhaltene Hohlfasermembran wird anschließend von einer Haspel aufgenommen und zu einer Fadenschar zusammengeführt. Aus der gehaspelten Fadenschar werden Hohlfasermembranbündel hergestellt. Nachfolgend wird die Porosität der Hohlfasermembran bestimmt.

Das Hohlfasermembranbündel wird nach bekannten Techniken zu Hohlfasermembranfiltern weiterverarbeitet, wie in der Messmethode 3 angegeben. Der erhaltene Hohlfasermembranfilter wird im folgenden Schritt mit einer Sterilisationsapparatur nach Beispiel 1 verbunden und der Hohlfasermembranfilter nach dem in Beispiel 1 geschilderten Verfahren sterilisiert. An sterilisierten Hohlfasermembranfiltern werden der Siebkoeffizient für ein Dextran mit einem Molekulargewicht von 10000g/ mol, das Zetapotential, der Siebkoeffizient für Albumin, der PVP Gehalt der Faser und der lokale wässrige Ultrafitrationskoeffizient an 5 verschiedenen Positionen im Hohlfasermembranfilter bestimmt. Die Ergebnisse sind in Tabelle 1 aufgelistet.

### Beispiel 3: Vergleichsbeispiel

Es werden gleiche Materialien wie in Beispiel 2 verwendet. Eine Spinnlösung bestehend aus 16 Gewichtsteilen Polysulfon, 4 Gewichtsteilen Polyvinylpyrrolidon und 80 Gewichtsteilen DMAC werden unter Rühren, Erwärmen auf 50°C und Entgasung zu einer homogenen Spinnmasse verarbeitet. Die Spinnmasse wird durch eine Ringspaltdüse mit einem zentral geführten Fällmittel, bestehend aus 54% DMAC und 46% Wasser, zu einem Spinnfaden extrudiert. Das Fällmittel wird im Inneren des hohlen Spinnfadens geführt. die Temperatur der Ringspaltdüse beträgt 40°C. Der extrudierte Spinnfaden wird durch einen Fällraum geführt, dessen Atmosphäre eine relative Feuchtigkeit von 30 % aufweist. Die Höhe des Fällspaltes beträgt 600mm, es wird eine Verweilzeit von 1,35 sec. im Fällspalt eingestellt. Der Spinnfaden wird in Fällbad, bestehend aus Wasser, eingeleitet, das auf 68°C temperiert ist und zu einer Hohlfasermembran ausgefällt. Anschließend wird die Hohlfasermembran durch Spülbäder, die auf eine Temperatur von 75°C bis 90°C temperiert sind, geleitet. Anschließend durchläuft die Hohlfasermembran einen Trocknungsprozess zwischen 100°C und 150°C. Die erhaltene Hohlfasermembran wird anschließend von einer Haspel aufgenommen und zu einer Fadenschar zusammengeführt. Aus der gehaspelten Fadenschar werden Hohlfasermembranbündel hergestellt. Nachfolgend wird die Porosität der Hohlfasermembran bestimmt.
Das Hohlfasermembranbündel wird nach bekannten Techniken zu einem Hohlfasermembranfilter weiterverarbeitet. Der erhaltene Hohlfasermembranfilter wird im folgenden Schritt nach einer im Stand der Technik beschriebenen Methode (DE 39 36 785 C1) sterilisiert. An sterilisierten Hohlfasermembranfiltern werden der Siebkoeffizient für ein Dextran mit einem Molekulargewicht von 10000g/mol, das Zetapotential, der Siebkoeffizient für Albumin, das Zetapotential, der PVP Gehalt, und die lokale Ultrafitrationen an 5 verschiedenen Positionen im Hohlfasermembranfilter bestimmt. Die Ergebnisse sind in der Tabelle 1 aufgelistet.

**Tabelle 1**

| **Ultrafiltrationskoeffizienten ermittelt an lokalen Positionen [ml/h*mmHg]** | | **Beispiel 2: Ausführungsbeispiel** | **Beispiel 3: Vergleichsbeispiel** |
|---|---|---|---|
| | Pos.1 | 137 | 119 |
| | Pos. 2 | 142 | 152 |
| | Pos. 3 | 148 | 154 |
| | Pos. 4 | 142 | 160 |
| | Pos. 5 | 144 | 150 |
| | Differenz min/ max | 11 | 35 |
| | Abweichung vom maximal Wert | 7,7% | 21,8% |
| **Zetapotential** | | -7 mV | -10 mV |
| **Porosität** | | 79,7% | 77% |
| **Siebkoeffizienten** Albumin | | 0,06 | 0,07 |
| **Siebkoeffizienten** Dextran (10000 g/mol) | | 0,63 | 0,40 |
| **Clearance Natrium** | | 268 | 260 |
| **Clearance Phosphat** | | 237 | 200 |
| **Clearance Vitamin B12** | | 169 | 146 |

Es werden die folgenden Ausführungen offenbart:

### Ausführung 1

Ein Verfahren zum Herstellen eines Hohlfasermembranbündels aus einer Vielzahl von auf Polysulfon und PVP basierenden Hohlfasermembranen, umfassend die Verfahrensschritte:
Bereitstellen einer Spinnlösung, umfassend ein Material auf Polysulfonbasis, insbesondere Polysulfon, ein Polymer auf Basis von Vinylpyrrolidon, insbesondere Polyvinylpyrrolidon, einem aprotischen Lösungsmittel, insbesondere Dimethylacetamid,
Bereitstellen einer Koagulationsflüssigkeit, umfassend Wasser und ein aprotisches Lösungsmittel, insbesondere Dimethylacetamid,
Koextrusion der Spinnlösung und der Koagulationsflüssigkeit durch eine konzentrische Ringspaltdüse zu einem hohlen Spinnfaden, wobei der Hohlraum des Spinnfadens mit koextrudierter Koagulationsflüssigkeit befüllt ist,
Durchleiten des Spinnfadens durch einen Fällspalt,
Einleiten des Spinnfadens in ein Fällbad, umfassend im Wesentlichen Wasser, um eine Hohlfasermembran zu erhalten,
Durchleiten der Hohlfasermembranen durch wenigstens ein Spülbad und Trocknen der erhaltenen Hohlfasermembran,
Anordnen der erhaltenen Hohlfasermembran zu einem Hohlfasermembranbündel,
Behandeln des Hohlfasermembranbündels mit Wasserdampf,
dadurch gekennzeichnet, dass die Behandlung mit Wasserdampf wenigstens einen Schritt umfasst, bei dem Wasserdampf in das Innere der Fasern geleitet wird und durch Druckbeaufschlagung durch die Membranwand auf die Außenseite der Fasern permeiert wird.

### Ausführung 2

Ein Verfahren zur Herstellung eines Hohlfasermembranbündels nach Ausführung 1, dadurch gekennzeichnet, dass die Spinnlösung 14% bis 18% Polysulfon und 3 bis 6% Polyvinylpyrrolidon enthält.

### Ausführung 3

Ein Verfahren zur Herstellung eines Hohlfasermembranbündels nach Ausführung 1 oder 2, dadurch gekennzeichnet, dass die Koagulationsflüssigkeit 25% bis 40% DMAC und 60% bis 75% Wasser umfasst.

### Ausführung 4

Ein Verfahren zur Herstellung eines Hohlfasermembranbündels nach einer der Ausführungen 1 bis 3, dadurch gekennzeichnet, dass die Spinndüse auf 70°C bis 85°C temperiert wird.

### Ausführung 5

Ein Verfahren zur Herstellung eines Hohlfasermembranbündels nach Ausführung 1 bis 4, dadurch gekennzeichnet, dass das Fällbad auf 70°C bis 90°C, insbesondere 75°C bis 90°C, temperiert wird.

### Ausführung 6

Ein Verfahren zur Herstellung eines Hohlfasermembranbündels nach einer der Ausführungen 1 bis 5, dadurch gekennzeichnet, dass die Hohlfasermembranen bei einer Temperatur von 75°C bis 90°C gewaschen werden.

### Ausführung 7

Ein Verfahren zur Herstellung eines Hohlfasermembranbündels nach einer der Ausführungen 1 bis 6, dadurch gekennzeichnet, dass die Hohlfasermembranen bei einer Temperatur von 100°C bis 150°C getrocknet werden.

### Ausführung 8

Ein Verfahren zum Sterilisieren eines Hohlfasermembranfilters mit einer Vielzahl von Hohlfasermembranen, die an den Enden so in dem Gehäuse des Hohlfasermembranfilters abgedichtet sind, dass ein erster Raum entsteht, der das Innere der Hohlfasermembranen umfasst und ein zweiter Raum entsteht, der einen Raum zwischen den Hohlfasermembranen umfasst, wobei der Hohlfasermembranfilter zumindest zwei Fluidzugänge aufweist, die mit dem ersten Raum verbunden sind und zumindest zwei Fluidzugänge aufweist, die mit dem zweiten Raum verbunden sind, und wobei die Fluidzugänge vorbereitet sind mit einer Sterilisationsvorrichtung verbunden zu werden, umfassend wenigstens die Schritte:
Spülen des Hohlfasermembranfilters mit einem Fluid, insbesondere Wasser, wobei das Spülfluid durch den ersten und den zweiten Raum des Hohlfasermembranfilters über eine Auswahl der Fluidzugänge geleitet wird,
Sterilisieren des Hohlfasermembranfilters mit einem sterilisierenden Fluid, insbesondere erhitztem Wasser oder Wasserdampf, wobei das sterilisierende Fluid durch den ersten und den zweiten Raum des Hohlfasermembranfilters über eine Auswahl der Fluidzugänge geleitet wird,
Zuführen eines Fluids, insbesondere Wasser oder Wasserdampf in den ersten oder den zweiten Raum des Hohlfasermembranfilters über eine Auswahl der Fluidzugänge,
   **und,**
transmembranes Überleiten des Fluids, insbesondere Wasser oder Wasserdampf, über die Membranwand hinweg in den jeweils zweiten oder ersten Raum der Hohlfasermembranfilters.

### Ausführung 9

Ein Verfahren nach Ausführung 8, dadurch gekennzeichnet, dass das Zuführen eines Fluids in den ersten oder zweiten Raum des Hohlfasermembranfilters und das transmembrane Überleiten des Fluids in den zweiten oder ersten Raum des Hohlfasermembranmoduls zwischen dem Spülvorgang und dem Sterilisationsvorgang erfolgt.

### Ausführung 10

Ein Verfahren nach Ausführung 8 oder 9, dadurch gekennzeichnet, dass es sich bei dabei um eine Hitzesterilisation, insbesondere eine Dampfsterilisation mit Wasserdampf, handelt.

### Ausführung 11

Ein Verfahren nach einer der Ausführungen 8 bis 10, dadurch gekennzeichnet, dass der Spülvorgang bei Temperaturen von 50°C bis 120°C stattfindet.

### Ausführung 12

Ein Verfahren nach einer der Ausführungen 8 bis 11, dadurch gekennzeichnet, dass die Hitzesterilisation bei Temperatur von 105°C bis 140°C, bevorzugt 121°C bis 140°C, durchgeführt wird.

### Ausführung 13

Ein Verfahren nach einer der Ausführungen 8 bis 12, dadurch gekennzeichnet, dass das Zuführen des Fluids in den ersten oder zweiten Raum des Hohlfasermembranfilters und das transmembrane Überleiten des Fluids in den jeweils zweiten oder ersten Raum des Hohlfasermembranmoduls bei Temperaturen von 70°C bis 98°C durchgeführt wird.

### Ausführung 14

Ein Verfahren nach einer oder mehreren der Ausführungen 8 bis 13, dadurch gekennzeichnet, dass ein weiterer Spülvorgang mit Druckgas, insbesondere steriler Druckluft erfolgt.

### Ausführung 15

Eine Hohlfasermembran, umfassend wenigstens ein Material auf Polysulfonbasis und wenigstens ein Polymer auf Basis von Polyvinylpyrrolidon, dadurch gekennzeichnet, dass die Hohlfasermembran eine Porosität von 77,5% bis 82% und einen Siebkoeffizienten für Dextran mit dem Molekulargewicht von 10.000 g/mol von 0,42 bis 0,75 aufweist.

### Ausführung 16

Eine Hohlfasermembran nach Ausführung 15, dadurch gekennzeichnet, dass die Hohlfasermembran einen Siebkoeffizienten für Albumin von kleiner als 0,01 aufweist.

### Ausführung 17

Eine Hohlfasermembran nach Ausführung 15 oder 16, dadurch gekennzeichnet, dass die Hohlfasermembran ein Zeta Potential von -3mV bis -10 mV aufweist.

### Ausführung 18

Eine Hohlfasermembran nach einer der Ausführungen 15 bis 17, dadurch gekennzeichnet, dass die Hohlfasermembran eine Porosität von 78% bis 81%, insbesondere eine Porosität von 79% bis 80,5% aufweist.

### Ausführung 19

Eine Hohlfasermembran nach einer der Ausführungen 15 bis 18, dadurch gekennzeichnet, dass die Hohlfasermembran einen Siebkoeffizienten für Dextran mit dem Molekulargewicht von 10.000 g/ mol von 0,45 bis 0,75, bevorzugt 0,55 bis 0,7, weiter bevorzugt 0,6 bis 0,7 aufweist.

### Ausführung 20

Eine Hohlfasermembran nach einer der Ausführung 15 bis 19, dadurch gekennzeichnet, dass die Hohlfasermembran einen Siebkoeffizienten für Albumin von kleiner als 0,005, insbesondere kleiner als 0,001 aufweist.

### Ausführung 21

Eine Hohlfasermembran nach mindestens einer der Ausführungen 15 bis 20, dadurch gekennzeichnet, dass die Hohlfasermembran ein Zeta-Potential von -4mV bis -8 mV, insbesondere von -6 mV bis -8 mV aufweist.

### Ausführung 22

Eine Hohlfasermembran nach einer der Ausführung 15 bis 21, dadurch gekennzeichnet, dass die Hohlfasermembran einen PVP Gehalt von 2,5 bis 5 Gew.% aufweist.

### Ausführung 23

Eine Hohlfasermembran nach einer der Ausführungen 15 bis 22, dadurch gekennzeichnet, dass die Hohlfasermembran auf der Innenseite nach XPS Analyse einen PVP Gehalt von 18% bis 27% aufweist.

### Ausführung 24

Eine Hohlfasermembran nach einer der Ausführungen 15 bis 23, dadurch gekennzeichnet, dass der Radius der nominelle Porengrößenverteilung im Bereich von 22 bis 26 Å (Angström), insbesondere im Bereich von 23 bis 26 Å (Angström) ein Maximum aufweist.

### Ausführung 25

Eine Hohlfasermembranfilter bestehend aus einem zylindrischen Gehäuse und einer Vielzahl von Hohlfasermembranen, die im Inneren des Gehäuses angeordnet sind und die an den Enden in einer Vergussmasse so abgedichtet sind, dass ein erster Raum entsteht, der den inneren Raum der Hohlfasermembranen umfasst und ein zweiter Raum entsteht, der den Raum zwischen den Hohlfasermembranen umfasst, wenigstens einem ersten Fluidzugang zur Zuleitung eines Fluids, insbesondere einer Flüssigkeit oder eines Gases in das Faserinnere, wenigstens einem ersten Fluidzugang zur Ableitung des Fluids, insbesondere der Flüssigkeit oder des Gases Flüssigkeit aus dem Faserinneren,
**dadurch gekennzeichnet,**
dass die Hohlfasermembranen über den Querschnitt des Hohlfasermembranfilters eine gleichmäßige Abtrennleistung aufweisen, die sich dadurch bemisst, dass die Hohlfasermembranen in unterschiedlichen Bereichen des Hohlfasermembranfilters einen Ultrafiltrationskoeffizienten aufweisen, die nicht mehr als 20% voneinander abweichen.

## Patentansprüche

1. Hohlfasermembran, umfassend wenigstens ein Material auf Polysulfonbasis und wenigstens ein Polymer auf Basis von Polyvinylpyrrolidon, **dadurch gekennzeichnet, dass** die Hohlfasermembran eine Porosität von 77,5% bis 82% und einen Siebkoeffizienten für Dextran mit dem Molekulargewicht von 10.000 g/mol von 0,42 bis 0,75 aufweist **dadurch gekennzeichnet, dass** die Hohlfasermembran einen Siebkoeffizienten für Albumin von kleiner als 0,01 aufweist, die Methoden zur Bestimmung der Porosität, des Dextran Siebkoeffizienten und des Albumin Siebkoeffizienten sind in der Beschreibung dargelegt.

2. Hohlfasermembran nach Anspruch1, **dadurch gekennzeichnet, dass** die Hohlfasermembran ein Zeta Potential von -3mV bis -10 mV aufweist, die Methode zur Bestimmung des Zetapotentials ist in der Beschreibung dargelegt.

3. Hohlfasermembran nach Anspruch 1 oder 2, **dadurch gekennzeichnet, dass** die Hohlfasermembran eine Porosität von 78% bis 81%, insbesondere eine Porosität von 79% bis 80,5% aufweist.

4. Hohlfasermembran nach einem der Ansprüche 1 bis 3, **dadurch gekennzeichnet, dass** die Hohlfasermembran einen Siebkoeffizienten für Dextran mit dem Molekulargewicht von 10.000 g/mol von 0,45 bis 0,75, bevorzugt 0,55 bis 0,7, weiter bevorzugt 0,6 bis 0,7 aufweist.

5. Hohlfasermembran nach einem der Ansprüche 1 bis 4, **dadurch gekennzeichnet, dass** die Hohlfasermembran einen Siebkoeffizienten für Albumin von kleiner als 0,005, insbesondere kleiner als 0,001 aufweist.

6. Hohlfasermembran nach mindestens einem der Ansprüche 1 bis 5, **dadurch gekennzeichnet, dass** die Hohlfasermembran ein Zeta-Potential von -4mV bis -8 mV, insbesondere von -6 mV bis -8 mV aufweist.

7. Hohlfasermembran nach einem der Ansprüche 1 bis 6, **dadurch gekennzeichnet, dass** die Hohlfasermembran einen PVP Gehalt von 2,5 bis 5 Gew.% aufweist.

8. Hohlfasermembran nach einem der Ansprüche 1 bis 7, **dadurch gekennzeichnet, dass** die Hohlfasermembran auf der Innenseite nach XPS Analyse einen PVP Gehalt von 18% bis 27% aufweist.

9. Hohlfasermembran nach einem der Ansprüche 1 bis 8, **dadurch gekennzeichnet, dass** der Radius der nominelle Porengrößenverteilung im Bereich von 22 bis 26 Å (Angström), insbesondere im Bereich von 23 bis 26 Å (Angström) ein Maximum aufweist, das Maximums des Radius der nominellen Porengrößenverteilung wird gemäß der Methode, die in der Beschreibung dargelegt ist ermittelt.
